# EUROPEAN PATENT APPLICATION

(11) **EP 4 467 571 A1**
(43) Date of publication of application: **27.11.2024**
(21) Application number: 23742866.9
(22) Date of filing: 17.01.2023
(51) Int. Cl.: C07K 16/46, C12N 15/13, A61K 39/395, A61K 38/19, A61P 35/00

(54) **ANTI-CD3 AND ANTI-CD20 BISPECIFIC ANTIBODY AND USE THEREOF**

(30) Priority: 20.01.2022 CN 202210068506
(71) Applicant: Shanghai Junshi Biosciences Co., Ltd., Pilot Free Trade Zone Shanghai 201210 (CN)
(72) Inventor: FENG, Hui, Suzhou, Jiangsu 215002 (CN); ZHOU, Yuehua, Suzhou, Jiangsu 215002 (CN); LIU, Dandan, Suzhou, Jiangsu 215002 (CN); LIU, Hongchuan, Suzhou, Jiangsu 215002 (CN); LI, Ruisheng, Suzhou, Jiangsu 215002 (CN); LIU, Hui, Suzhou, Jiangsu 215002 (CN); HE, Zhijuan, Suzhou, Jiangsu 215002 (CN)
(74) Representative: Cabinet Becker et Associés
(86) International application number: PCT/CN2023/072510
(87) International publication number: WO 2023/138551

(57) **Abstract**

The present invention provides a bispecific antibody targeting CD3 and CD20 and a composition comprising same. Further provided are a nucleic acid molecule encoding the bispecific antibody of the present invention, a vector and a host cell for expressing the bispecific antibody of the present invention, and a therapeutic and diagnostic method and a use of the antibody of the present invention or an antigen-binding fragment thereof.

## Description

### TECHNICAL FIELD

The present invention relates to the field of antibodies, and in particular to a bispecific antibody targeting CD3 and CD20 and a composition comprising the same. The present invention also relates to a nucleic acid molecule encoding the antibody or the antigen-binding fragment thereof of the present invention, a vector and a host cell used for expressing the antibody or the antigen-binding fragment thereof of the present invention, and a therapeutic and diagnostic method and use of the antibody or the antigen-binding fragment thereof of the present invention.

### BACKGROUND

CD3 (T cell surface glycoprotein CD3, a signaling co-receptor for the T cell receptor, which comprises subunits γ, δ, ε, and ζ) is a differentiation antigen expressed on the surface of all T lymphocytes and primarily mediates the transduction of T cell activation signals. CD3 plays an important role in anti-infection immunity of the body immune system. The CD3 molecule forms a stable TCR-CD3 complex with a T cell antigen receptor (T cell receptor). The extracellular domain of the CD3 complex recognizes and binds to a major histocompatibility complex II molecule, which enhances the binding stability of the T cell antigen receptor (T cell receptor, TCR) to the MHC molecule; the intracellular domain enhances the activation signal transduced by leukocyte CD3, thereby participating in and regulating the activation of the immune system. The number of the CD3-positive lymphocyte population is an important index for measuring the cellular immunity condition of the body.

CD20 is a marker molecule specific for the surface of B lymphocytes and is expressed on mature B cells and most malignant B lymphocytes, but not on early progenitor B cells or late mature plasma cells. The molecule consists of 297 amino acid residues, penetrates the cell membrane four times, and has an antigenic epitope which is the only ring exposed outside the cell and consists of 43 amino acid residues without sugar chains in the third transmembrane region and the fourth transmembrane region. The exact function of CD20 is still unclear, but CD20 may be involved in the activation and differentiation of B cells and function as a calcium channel. Since CD20 is not absorbed into cells and does not undergo significant shedding from the cell surface after binding to the antibody, it is an ideal antigen for treating B lymphocyte-related diseases. For example, rituximab and obinutuzumab are both antibody drugs targeting CD20 and have good efficacy. Given that existing antibodies against CD20 still have room to rise in mediating the killing effect of immune cells on tumor cells and the advantages of bispecific antibodies, good anti-CD20/CD3 bispecific antibodies can well mediate the killing effect of immune cells on tumor cells, which is very promising for clinical application.

Bispecific antibody (BsAb), also known as a bifunctional antibody, can specifically bind to two different antigens or two different epitopes simultaneously. Due to the specificity and bifunctionality, the bispecific antibody has good application effects and prospects in the fields of tumor immunotherapy, autoimmune disease, and the like. The bispecific antibody has various forms, and the effects of different bispecific antibody forms are different for different tumor-associated antigens. Because of this special function, it has broad application prospects in tumor immunotherapy.

For the anti-CD3×CD20 bispecific antibody, there are currently no drugs on the market. The present invention aims to develop a novel bispecific antibody with good effects in terms of affinity, safety, stability, and the like.

### SUMMARY

Disclosed herein is a novel bispecific antibody molecule constructed by an antibody engineering method.

The present invention provides a bispecific antibody specifically binding to CD20 and CD3, comprising:
(1) an antigen-binding fragment having a CD20 binding domain, comprising a first heavy chain variable region (VH1) and a first light chain variable region (VL1); and
(2) an antigen-binding fragment having a CD3 binding domain, comprising a second heavy chain variable region (VH2) and a second light chain variable region (VL2).

In some embodiments, provided is the bispecific antibody described herein, comprising the following polypeptide chains:
(1) polypeptide chains of formula (I) and formula (II) having a CD20 binding domain:

   VH1-CH1-Fc1 formula (I),

   and

   VL1-CL formula (II);

   and
(2) polypeptide chains of formula (III) and formula (II-2) having CD20 and CD3 binding domains:

   VH1-CH1-L-VL2-L-VH2-L-Fc2 formula (III),

   and

   VL1-CL formula (II-2);

   or
   a polypeptide chain of formula (IV) having a CD3 binding domain:

   VL2-L-VH2-L-Fc2 formula (IV);

wherein VH represents a heavy chain variable region; VL represents a light chain variable region; Fc comprises CH2 and CH3; CH1, CH2, and CH3 represent domains 1, 2, and 3 of the heavy chain constant region, respectively; VL represents a light chain variable region; CL represents a light chain constant region; L represents a linker peptide, the linker peptides being identical or different; optionally, a hinge region is present between CH1 and Fc1;
formulas (I), (II), (II-2), (III), and (IV) are each formed in a sequential connection from N-terminus to C-terminus;
an antigen-binding site formed by VH1 and VL1 binds to CD20, and an antigen-binding site formed by VL2 and VH2 binds to CD3.

In some embodiments, the linker peptide described herein comprises an amino acid sequence (GGGGS)n, wherein n is each independently selected from 1, 2, 3, 4, 5, or 6.

In some embodiments, VH1 described herein comprises HCDR1, HCDR2, and HCDR3 having amino acid sequences set forth in SEQ ID NO: 1, SEQ ID NO: 2, and SEQ ID NO: 3, respectively, and VL1 described herein comprises LCDR1, LCDR2, and LCDR3 having amino acid sequences set forth in SEQ ID NO: 4, SEQ ID NO: 5, and SEQ ID NO: 6, respectively.

In some embodiments, provided is the bispecific antibody described herein, wherein:
VH1 comprises an amino acid sequence set forth in SEQ ID NO: 7, or an amino acid sequence having at least 90%, 91%, 92%, 93%, 94%, 95%, 96%, 97%, 98%, or 99% identity to the amino acid sequence set forth in SEQ ID NO: 7; and
VL1 comprises an amino acid sequence set forth in SEQ ID NO: 8; or comprises an amino acid sequence having at least 90%, 91%, 92%, 93%, 94%, 95%, 96%, 97%, 98%, or 99% identity to the amino acid sequence set forth in SEQ ID NO: 8.

In some embodiments, provided is the bispecific antibody described herein, wherein VH2 comprises HCDR1, HCDR2, and HCDR3 having amino acid sequences set forth in SEQ ID NO: 9, SEQ ID NO: 10, and SEQ ID NO: 11, respectively, and VL2 comprises LCDR1, LCDR2, and LCDR3 having amino acid sequences set forth in SEQ ID NO: 12, SEQ ID NO: 13, and SEQ ID NO: 14, respectively.

In some embodiments, provided is the bispecific antibody described herein, wherein:
VH2 comprises an amino acid sequence set forth in SEQ ID NO: 15, or an amino acid sequence having at least 90%, 91%, 92%, 93%, 94%, 95%, 96%, 97%, 98%, or 99% identity to the amino acid sequence set forth in SEQ ID NO: 15; and
VL2 comprises an amino acid sequence set forth in SEQ ID NO: 16, or an amino acid sequence having at least 90%, 91%, 92%, 93%, 94%, 95%, 96%, 97%, 98%, or 99% identity to the amino acid sequence set forth in SEQ ID NO: 16.

In some embodiments, provided is the bispecific antibody described herein, wherein:
the polypeptide chain of formula (I) comprises an amino acid sequence set forth in SEQ ID NO: 17 or 23, or an amino acid sequence having at least 90%, 91%, 92%, 93%, 94%, 95%, 96%, 97%, 98%, or 99% identity to the amino acid sequence set forth in SEQ ID NO: 17 or 23; and
the polypeptide chains of formulas (II) and (II-2) comprise an amino acid sequence set forth in SEQ ID NO: 18, or an amino acid sequence having at least 90%, 91%, 92%, 93%, 94%, 95%, 96%, 97%, 98%, or 99% identity to the amino acid sequence set forth in SEQ ID NO: 18.

In some embodiments, provided is the bispecific antibody described herein, wherein the polypeptide chain of formula (III) comprises an amino acid sequence set forth in SEQ ID NO: 19, or an amino acid sequence having at least 90%, 91%, 92%, 93%, 94%, 95%, 96%, 97%, 98%, or 99% identity to the amino acid sequence set forth in SEQ ID NO: 19.

In some embodiments, provided is the bispecific antibody described herein, wherein the polypeptide chain of formula (IV) comprises an amino acid sequence set forth in SEQ ID NO: 20, or an amino acid sequence having at least 90%, 91%, 92%, 93%, 94%, 95%, 96%, 97%, 98%, or 99% identity to the amino acid sequence set forth in SEQ ID NO: 20.

In some embodiments, provided is the bispecific antibody described herein, which comprises polypeptide chains of formula (I) and formula (II), and polypeptide chains of formula (III) and formula (II-2), wherein the polypeptide chain of formula (I) comprises an amino acid sequence set forth in SEQ ID NO: 23, the polypeptide chains of formula (II) and formula (II-2) comprise amino acid sequences set forth in SEQ ID NO: 18, and the polypeptide chain of formula (III) comprises an amino acid sequence set forth in SEQ ID NO: 19.

In some embodiments, provided is the bispecific antibody described herein, which comprises polypeptide chains of formula (I) and formula (II), and a polypeptide chain of formula (IV), wherein the polypeptide chain of formula (I) comprises an amino acid sequence set forth in SEQ ID NO: 17, the polypeptide chain of formula (II) comprises an amino acid sequence set forth in SEQ ID NO: 18, and the polypeptide chain of formula (IV) comprises an amino acid sequence set forth in SEQ ID NO: 20.

In some embodiments, provided is the bispecific antibody described herein, wherein formula (I) and formula (II) are connected via a disulfide bond, and formula (III) and formula (II-2) are connected via a disulfide bond; and formula (I) and formula (III) or formula (IV) are connected via a disulfide bond and a Knob into Hole structure of the CH3 domain;
preferably, Fc1 in formula (I) is knob-Fc, and Fc2 in formula (III) or formula (IV) is hole-Fc; or Fc1 in formula (I) is hole-Fc, and Fc2 in formula (III) or formula (IV) is knob-Fc.

In some embodiments, provided is the bispecific antibody described herein, wherein CH1-Fc1 in formula (I) and Fc2 in formula (III) are in the form of IgG, e.g., IgG1, IgG2, IgG3, or IgG4, and/or CL in formula (II) and formula (II-2) is derived from a λ or κ chain.

In yet another aspect, the present invention provides an isolated nucleic acid encoding any one or more of the polypeptide chains in the bispecific antibody described herein.

In yet another aspect, the present invention provides an expression vector comprising the nucleic acid described herein; preferably, the expression vector is a eukaryotic expression vector.

In yet another aspect, the present invention provides a host cell comprising the nucleic acid described herein or the expression vector described herein; preferably, the host cell is a eukaryotic cell; more preferably, the host cell is a mammalian cell.

In yet another aspect, the present invention provides a method for preparing the bispecific antibody described herein, comprising culturing the host cell described herein under conditions suitable for the expression of the nucleic acid described herein, and isolating the bispecific antibody from the host cell.

In yet another aspect, the present invention provides a pharmaceutical composition, which comprises the bispecific antibody described herein, the polynucleotide described herein, the expression vector described herein, and/or the host cell described herein, and a pharmaceutically acceptable carrier or excipient.

In yet another aspect, the present invention provides use of the bispecific antibody described herein, the polynucleotide described herein, the expression vector described herein, the host cell described herein, and/or the pharmaceutical composition described herein in the preparation of a medicament for preventing or treating cancer; preferably, the cancer is selected from acute B-lymphocytic leukemia, diffuse large B-cell lymphoma, chronic lymphocytic leukemia, follicular lymphoma, non-Hodgkin's lymphoma, chronic myelocytic leukemia, or Burkitt's lymphoma.

In yet another aspect, the present invention provides a method for preventing or treating cancer in a subject, which comprises administering to a subject in need thereof the bispecific antibody described herein, the polynucleotide described herein, the expression vector described herein, the host cell described herein, and/or the pharmaceutical composition described herein.

In yet another aspect, the present invention provides the bispecific antibody described herein, the polynucleotide described herein, the expression vector described herein, the host cell described herein, and/or the pharmaceutical composition described herein, for use in the treatment of cancer.

In some embodiments, the cancer described herein is selected from acute B-lymphocytic leukemia, diffuse large B-cell lymphoma, chronic lymphocytic leukemia, follicular lymphoma, non-Hodgkin's lymphoma, chronic myelocytic leukemia, or Burkitt's lymphoma.

In yet another aspect, the present invention provides a drug combination comprising the antibody or the antigen-binding fragment thereof described herein, the polynucleotide described herein, the expression vector described herein, the host cell described herein, and/or the pharmaceutical composition described herein, and one or more additional therapeutic agents.

In yet another aspect, the present invention provides a method for detecting the presence of CD3 and/or CD20 in a sample using the bispecific antibody described herein.

In yet another aspect, the present invention provides a kit comprising the antibody described herein or the pharmaceutical composition described herein.

### BRIEF DESCRIPTION OF THE DRAWINGS

FIG. 1: schematic diagram of the molecular structure of an anti-CD3×CD20 bispecific antibody, 1a: schematic structural diagram of TZT6; 1b: schematic structural diagram of TZT7.
FIG. 2: assay of binding of bispecific antibodies to human CD3ε by ELISA.
FIG. 3: binding of bispecific antibodies to Raji cells.
FIG. 4: binding of bispecific antibodies to Jurkat cells.
FIG. 5: binding of bispecific antibodies to cells overexpressing cynomolgus monkey CD3e.
FIG. 6: activity of bispecific antibodies by a luciferase reporter assay.
FIG. 7: activation activity of bispecific antibodies for T lymphocytes.
FIG. 8: activity of bispecific antibodies for promoting the killing of B lymphoma cells by T cells.
FIG. 9: inhibition of growth of B16 OVA huCD20 tumor by bispecific antibodies TZT6 and TZT7.
FIG. 10: inhibition of growth of B16 OVA huCD20 tumor by a bispecific antibody TZT7.
FIG. 11: inhibitory effect of bispecific antibodies on the Raji Mixeno model of human lymphoma.

### DETAILED DESCRIPTION

### Definitions

Unless otherwise stated, embodiments of the present invention will employ conventional techniques of molecular biology (including recombinant techniques), microbiology, cytobiology, biochemistry, and immunology, which are all within the skill of the art.

In order to facilitate the understanding of the present invention, some technical and scientific terms are specifically defined as follows. Unless otherwise specifically defined herein, all technical and scientific terms used herein have the same meaning as commonly understood by those of ordinary skill in the art to which the present invention belongs. For definitions and terminology in the art, the skilled person can refer specifically to Current Protocolsin Molecular Biology (Ausubel). Abbreviations for amino acid residues are standard 3-letter and/or 1-letter codes used in the art to denote one of the 20 commonly used L-amino acids. The singular forms used herein (including claims) include their corresponding plural forms, unless otherwise explicitly specified herein.

The term "about" used in combination with a numerical value is intended to encompass the numerical values in a range from a lower limit less than the specified numerical value by 5% to an upper limit greater than the specified numerical value by 5%.

The term "and/or" should be understood as any one of the options or a combination of any two or more of the options.

The term "CD3" refers to a part of the T cell receptor complex, which consists of three different chains, CD3ε, CD3δ, and CD3γ. The concentration of CD3 on T cells through, for example, its immobilization by anti-CD3 antibodies, results in T cell activation, which is similar to T cell receptor-mediated activation, but independent of the specificity of TCR clones. Most anti-CD3 antibodies recognize the CD3ε chain. The term refers to any natural CD3 from any vertebrate (including mammals such as primates (e.g., humans)) and rodents (e.g., mice and rats), unless otherwise stated. This term encompasses "full-length" unprocessed CD3 and CD3 in any form resulting from intracellular processing or any fragment thereof. The term also includes variants of naturally occurring CD3, e.g., splice variants or allelic variants. In a preferred embodiment, CD3 refers to full-length CD3 from humans and cynomolgus monkeys or fragments thereof (such as mature fragments thereof lacking a signal peptide). In a preferred embodiment, CD3 refers to full-length CD3 from mice/rats or fragments thereof (such as mature fragments thereof lacking a signal peptide).

The term "human CD20" or "CD20" refers to human CD20 (UniProtKB/Swiss-Prot No. P11836) and includes
any variant, isoform and species homolog of CD20 naturally expressed by cells (including tumor cells) or expressed on cells transfected with a CD20 gene or cDNA. Species homologs include rhesus monkey CD20 (macaca mulatta; UniProtKB/Swiss-Prot No H9YXP1) and cynomolgus monkey CD20.

The term "percent (%) amino acid sequence identity", or simply "identity", is defined as the percentage of amino acid residues in a candidate amino acid sequence that are identical to those in a reference amino acid sequence after aligning the amino acid sequences (with gaps introduced if necessary) to achieve maximum percent sequence identity without considering any conservative substitution as part of sequence identity. Various methods in the art can be employed to perform sequence alignment so as to determine the percent amino acid sequence identity, for example, using computer software available to the public, such as BLAST, BLAST-2, ALIGN, or MEGALIGN (DNASTAR) software. Those skilled in the art can determine suitable parameters for measuring alignment, including any algorithm required to obtain maximum alignment for the full length of the aligned sequences.

The term "immune response" refers to the action of, for example, lymphocytes, antigen-presenting cells, phagocytes, granulocytes, and soluble macromolecules produced by the above cells or liver (including antibodies, cytokines and complements) that result in selective damage to, destruction of, or elimination from the human body of invading pathogens, cells or tissues infected with pathogens, cancerous cells, or, in the cases of autoimmunity or pathological inflammation, normal human cells or tissues.

The term "signal transduction pathway" or "signal transduction activity" refers to a biochemical causal relationship generally initiated by an interaction between proteins (such as binding of a growth factor to a receptor) and resulting in the transmission of a signal from one portion of a cell to another portion of the cell. In general, the transmission includes specific phosphorylation of one or more tyrosine, serine, or threonine residues on one or more proteins in a series of reactions causing signal transduction. The penultimate process typically involves a nuclear event, resulting in a change in gene expression.

The term "activity" or "bioactivity", or the term "biological property" or "biological characteristic" can be used interchangeably herein and includes, but is not limited to, epitope/antigen affinity and specificity, the ability to neutralize or antagonize the activity of CD20 *in vivo* or *in vitro,* IC50, the *in vivo* stability of the antibody, and the immunogenic properties of the antibody. Other identifiable biological properties or characteristics of the antibody known in the art include, for example, cross-reactivity (i.e., cross-reactivity with non-human homologs of the targeted peptide, or with other proteins or tissues in general), and the ability to maintain a high expression level of the protein in mammalian cells. The aforementioned properties or characteristics are observed, determined, or assessed using techniques well known in the art, including but not limited to ELISA, FACS, or BIACORE plasma resonance analysis, unlimited *in vitro* or *in vivo* neutralization assays, receptor binding, cytokine or growth factor production and/or secretion, signal transduction, and immunohistochemistry of tissue sections of different sources (including human, primate or any other source).

The term "antibody" refers to any form of an antibody with the desired bioactivity. Thus, it is used in the broadest sense and specifically includes, but is not limited to, monoclonal antibodies (including full-length monoclonal antibodies), polyclonal antibodies, multispecific antibodies (e.g., bispecific antibodies), humanized antibodies, fully human antibodies, chimeric antibodies, and camelized single-domain antibodies.

The term "isolated antibody" refers to the purified state of a binding compound, and, in this case, means that the molecule is substantially free of other biomolecules, such as nucleic acids, proteins, lipids, sugars, or other substances such as cell debris and growth medium. The term "isolate(d)" does not mean the complete absence of such substances or the absence of water, buffers, or salts, unless they are present in amounts that will significantly interfere with the experimental or therapeutic use of the binding compounds described herein.

The term "monoclonal antibody" refers to an antibody obtained from a substantially homogeneous population of antibodies, i.e., the antibodies composing the population are identical except for possible naturally occurring mutations that may be present in minor amounts. A monoclonal antibody is highly specific and targets a single antigen epitope. In contrast, conventional (polyclonal) antibody preparations typically include a large number of antibodies targeting (or specific for) different epitopes. The modifier "monoclonal" indicates the characteristic of an antibody obtained from a substantially homogeneous population of antibodies, and is not to be construed as producing the antibody by any particular method.

The term "bispecific antibody" refers to an antibody molecule capable of binding to two separate antigens or having binding specificity for different epitopes within the same antigen. For example, in some embodiments, one arm of the bispecific antibody molecule binds to a tumor-associated antigen and the other arm binds to an immune cell-associated antigen (e.g., a CD3 molecule), which can activate and initiate cellular immunity-related mechanisms at the tumor cell.

The term "full-length antibody" refers to an immunoglobulin molecule comprising four peptide chains when occurring naturally: two heavy (H) chains (about 50-70 kDa in full length) and two light (L) chains (about 25 kDa in full length) connected to each other via disulfide bonds. Each of the heavy chains consists of a heavy chain variable region (abbreviated herein as VH) and a heavy chain constant region (abbreviated herein as CH). The heavy chain constant region consists of 3 domains, i.e., CH1, CH2, and CH3. Each of the light chains consists of a light chain variable region (abbreviated herein as VL) and a light chain constant region. The light chain constant region consists of one domain CL. The VH and VL regions can be further divided into complementarity-determining regions (CDRs) with high variability and more conservative regions called framework regions (FRs) that are spaced apart by the CDRs. Each VH or VL region consists of 3 CDRs and 4 FRs arranged in the following order from the amino terminus to the carboxyl terminus: FR1, CDR1, FR2, CDR2, FR3, CDR3, and FR4. The variable regions of the heavy and light chains contain binding domains that interact with antigens. The constant regions of an antibody can mediate the binding of immunoglobulins to host tissues or factors, including various cells of the immune system (e.g., effector cells) and the first component (C1q) of classical complement system.

The term "antigen-binding fragment" of an antibody ("parent antibody") includes a fragment or a derivative of the antibody, generally including at least one fragment of an antigen-binding region or variable region (e.g., one or more CDRs) of a parent antibody, which retains at least some of the binding specificity of the parent antibody. Examples of the binding fragment of an antibody include, but are not limited to, Fab, Fab', F(ab')₂, and Fv fragments; a diabody; a linear antibody; a single-chain antibody molecule, e.g., sc-Fv; a nanobody and a multispecific antibody formed by fragments of the antibody. The binding fragment or the derivative generally retains at least 10% of the antigen-binding activity of the parent antibody when the antigen-binding activity is expressed on a molar concentration basis. Preferably, the binding fragment or the derivative retains at least 20%, 50%, 70%, 80%, 90%, 95% or 100% or more of the antigen binding affinity of the parent antibody. It is also contemplated that the antigen-binding fragment of the antibody may include conservative or non-conservative amino acid substitutions that do not significantly alter its bioactivity (referred to as "conservative variants" or "function-conservative variants" of the antibody). The term "binding compound" refers to both the antibody and the binding fragment thereof.

The term "single-chain Fv" or "scFv" antibody refers to an antibody fragment comprising the VH and VL domains of an antibody, where these domains are present in a single polypeptide chain. In general, an Fv polypeptide also comprises a polypeptide linker between the VH and VL domains that enables the scFv to form the desired structure for antigen binding.

The term "Fc", "Fc region", or "Fc fragment" refers to a polypeptide consisting of CH2 and CH3 domains of IgA, IgD, and IgG, or CH2, CH3, and CH4 domains of IgE and IgM, via a hinge region. Although the breakdown of the Fc fragment is variable, the heavy chain Fc fragment of human IgG is generally referred to as a polypeptide from A231 to its carboxyl terminus.

The term "hinge region" refers to the proline-rich, flexible polypeptide chain between CH1 and CH2 in an antibody. The recognized IgG hinge region is a polypeptide chain consisting of amino acid residues from position 216 to 230.

The term "domain antibody" is an immunofunctional immunoglobulin fragment that contains only the heavy chain variable region or the light chain variable region. In certain cases, two or more VH regions are covalently linked to a peptide linker to form a bivalent domain antibody. The two VH regions of the bivalent domain antibody may target the same or different antigens.

The term "epitope" refers to a protein determinant capable of specifically binding to an antibody. Epitopes are usually composed of molecules clustered on the surface, such as amino acids or sugar side chains, and usually have specific three-dimensional structural characteristics, as well as specific charge characteristics. Conformational and non-conformational epitopes differ in that binding to the former, but not the latter, is lost in the presence of denaturing solvents. The epitopes may comprise amino acid residues directly involved in the binding and other amino acid residues not directly involved in the binding, e.g., amino acid residues effectively blocked or covered by the specific antigen-binding peptide (in other words, amino acid residues within the footprint of the specific antigen-binding peptide).

The term "bivalent antibody" comprises two antigen-binding sites. In certain cases, the two binding sites have the same antigen specificity. However, the bivalent antibody may be bispecific.

The term "diabody" refers to a small antibody fragment having two antigen-binding sites and comprising a heavy chain variable domain (VH) linked to a light chain variable domain (VL) in the same polypeptide chain (VH-VL or VL-VH). By using a linker that is too short to allow pairing between two domains in one chain, the domains are forced to pair with the complementary domains of the other chain to form two antigen-binding sites.

The term "chimeric antibody" is an antibody having variable domains of a first antibody and constant domains of a second antibody, wherein the first and second antibodies are from different species. Typically, the variable domains are obtained from an antibody of an animal such as a rodent ("parent antibody"), and the constant domain sequences are obtained from a human antibody, such that the resulting chimeric antibody is less likely to induce an adverse immune response in a human subject as compared to the parent rodent antibody.

The term "humanized antibody" refers to an antibody form containing sequences from both human and non-human (such as mouse and rat) antibodies. In general, the humanized antibody comprises substantially all of at least one, and typically two, variable domains, in which all or substantially all of the hypervariable loops are equivalent to those of a non-human immunoglobulin and all or substantially all of the framework regions (FRs) are those of a human immunoglobulin sequence. The humanized antibody may optionally comprise at least a portion of a human immunoglobulin constant region (Fc).

The term "fully human antibody" refers to an antibody that comprises only human immunoglobulin sequences. A fully human antibody may contain mouse glycochains if produced in mice, mouse cells or hybridomas derived from mouse cells. Likewise, "a mouse antibody" refers to an antibody that comprises only mouse immunoglobulin sequences. Alternatively, a fully human antibody may contain rat glycochains if produced in rats, rat cells or hybridomas derived from rat cells. Likewise, a "rat antibody" refers to an antibody that comprises only rat immunoglobulin sequences.

"Isotypes" of antibodies refer to types of antibodies (e.g., IgM, IgE and IgG (such as IgG1, IgG2 or IgG4)) provided by heavy chain constant region genes. Isotype also includes modified forms of one of these types in which modifications have been made to alter Fc function, for example, to enhance or attenuate effector function or binding to Fc receptors.

The term "epitope" refers to the region of an antigen to which an antibody binds. Epitopes can be formed from contiguous amino acids or noncontiguous amino acids juxtaposed by tertiary folding of a protein.

"Affinity" or "binding affinity" refers to inherent binding affinity that reflects the interaction between members of a binding pair. The affinity of molecule X for its partner Y can be generally represented by the equilibrium dissociation constant (KD), which is a ratio of the dissociation rate constant to the association rate constant (kdis and kon, respectively). Affinity can be measured using common methods known in the art. One specific method for measuring affinity is the ForteBio kinetic binding assay herein.

The term "not bind to" a protein or cell means not binding to the protein or cell, or not binding to it with high affinity, that is, binding to the protein or cell with a KD of 1.0 × 10⁻⁶ M or higher, more preferably 1.0 × 10⁻⁵ M or higher, more preferably 1.0 × 10⁻⁴ M or higher, 1.0 × 10⁻³ M or higher, and more preferably 1.0 × 10⁻² M or higher.

The term "high affinity" for IgG antibodies means a KD for the antigen of 1.0 × 10⁻⁶ M or less, preferably 5.0 × 10⁻⁸ M or less, more preferably 1.0 × 10⁻⁸ M or less, 5.0 × 10⁻⁹ M or less, and more preferably 1.0 × 10⁻⁹ M or less. For other antibody subtypes, "high affinity" binding may vary. For example, "high affinity" binding of the IgM subtype refers to a KD of 10⁻⁶ M or less, preferably 10⁻⁷ M or less, and more preferably 10⁻⁸ M or less.

The term "antibody-dependent cytotoxicity", "antibody-dependent cell-mediated cytotoxicity", or "ADCC" refers to a cell-mediated immune defense in which the effector cells of the immune system actively lyse target cells, such as cancer cells, whose cell membrane surface antigens bind to antibodies, such as a Claudin18.2 antibody.

The term "complement-dependent cytotoxicity" or "CDC" refers to the effector function of IgG and IgM antibodies, which, when binding to surface antigens, trigger typical complement pathways, including the formation of membrane attack complexes and lysis of target cells. The antibody of the present invention, when binds to CD20, triggers CDC against cancer cells.

The term "nucleic acid" or "polynucleotide" refers to deoxyribonucleic acid (DNA) or ribonucleic acid (RNA) and polymers thereof in either single- or double-stranded form. Unless explicitly limited, the term includes nucleic acids containing known analogs of natural nucleotides that have binding properties similar to that of the reference nucleic acid and are metabolized in a manner similar to naturally occurring nucleotides (see U.S. Pat. No. 8,278,036 to Kariko et al., which discloses an mRNA molecule with uridine replaced by pseudouridine, a method for synthesizing the mRNA molecule, and a method for delivering a therapeutic protein *in vivo*). Unless otherwise specified, a particular nucleic acid sequence also implicitly includes conservatively modified variants thereof (e.g., degenerate codon substitutions), alleles, orthologs, SNPs, and complementary sequences as well as the sequence explicitly indicated. Specifically, degenerate codon substitutions can be achieved by generating sequences in which the third position of one or more selected (or all) codons is substituted with mixed bases and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

"Construct" refers to any recombinant polynucleotide molecule (such as plasmid, cosmid, virus, autonomously replicating polynucleotide molecule, phage, or linear or circular single- or double-stranded DNA or RNA polynucleotide molecule) derived from any source, capable of genomic integration or autonomous replication, and comprising a polynucleotide molecule where one or more polynucleotide molecules have been linked in a functionally operative manner (i.e., operably linked). The recombinant construct typically comprises a polynucleotide of the present invention operably linked to transcription initiation regulatory sequences that will direct transcription of the polynucleotide in a host cell. Both heterologous and non-heterologous (i.e., endogenous) promoters can be used to direct expression of the nucleic acids of the present invention.

"Vector" refers to any recombinant polynucleotide construct that can be used for transformation purpose (i.e., the introduction of heterologous DNA into a host cell). One type of vector is a "plasmid", which refers to a circular double-stranded DNA loop into which additional DNA segments can be ligated. Another type of vector is a viral vector, in which additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of the host cell upon introduction into a host cell, and are thereby replicated along with the host genome. In addition, certain vectors are capable of directing the expression of operably linked genes. Such vectors are referred to herein as "expression vectors".

The term "expression vector" as used herein refers to a nucleic acid molecule capable of replicating and expressing a target gene when transformed, transfected or transduced into a host cell. The expression vector comprises one or more phenotypic selectable markers and an origin of replication to ensure the maintenance of the vector and to provide amplification in the host if needed.

Unless otherwise or explicitly specified in the context, "activation", "stimulation" and "treatment" for a cell or a receptor may have the same meaning. For example, the cell or the receptor is activated, stimulated or treated with a ligand. "Ligands" include natural and synthetic ligands, such as cytokines, cytokine variants, analogs, mutant proteins, and binding compounds derived from antibodies. "Ligands" also include small molecules, such as peptidomimetics of cytokines and peptidomimetics of antibodies. "Activation" may refer to the activation of a cell regulated by internal mechanisms and external or environmental factors. "Response/reaction", e.g., a response of a cell, a tissue, an organ or an organism, includes changes in biochemical or physiological behaviors (e.g., concentration, density, adhesion or migration, gene expression rate, or differentiation state within a biological compartment), where the changes are associated with activation, stimulation or treatment, or are associated with an internal mechanism such as genetic programming.

As used herein, the term "treat", "treating" or "treatment" of any disease or disorder refers in one embodiment to ameliorating the disease or disorder (i.e., slowing or arresting or reducing the progression of the disease or at least one of its clinical symptoms). In another embodiment, "treat", "treating" or "treatment" refers to alleviating or ameliorating at least one physical parameter, including those physical parameters that may not be discernible by the patient. In another embodiment, "treat", "treating" or "treatment" refers to modulating the disease or disorder, physically (e.g., stabilization of discernible symptoms), physiologically (e.g., stabilization of physical parameters), or both. Unless explicitly described herein, methods for assessing treatment and/or prevention of disease are generally known in the art.

A "subject" includes any human or non-human animal. The term "non-human animal" includes all vertebrates, e.g., mammals and non-mammals, such as non-human primates, sheep, dogs, cats, horses, cattle, chickens, amphibians, and reptiles. As used herein, the term "cyno" refers to a cynomolgus monkey.

Administration "in combination with" one or more other therapeutic agents includes simultaneous (co-) administration and sequential administration in any order.

"Therapeutically effective amount", "therapeutically effective dose" and "effective amount" refer to an amount of the anti-CD20 antibody or the antigen-binding fragment thereof of the present invention that is effective in preventing or ameliorating one or more symptoms of a disease or condition or the progression of the disease or condition when administered alone or in combination with other therapeutic drugs to a cell, a tissue or a subject. The therapeutically effective dose also refers to an amount of the antibody or the antigen-binding fragment thereof sufficient to cause amelioration of symptoms, e.g., an amount for treating, curing, preventing or ameliorating a related condition or promoting the treatment, cure, prevention or amelioration of such condition. When an active ingredient is administered to an individual alone, a therapeutically effective dose refers to the amount of the ingredient. In the case of administration in combination, a therapeutically effective dose refers to the combined amount of active ingredients that produces a therapeutic effect, regardless of whether these active ingredients are administered in combination, sequentially or simultaneously. An effective amount of a therapeutic agent will increase a diagnostic index or parameter by at least 10%, generally at least 20%, preferably at least about 30%, more preferably at least 40%, and most preferably at least 50%.

"Cancer" and "cancerous" refer to or describe the physiological condition in mammals which is typically characterized by unregulated cell growth. Included in this definition are benign and malignant cancers as well as dormant tumors or micrometastases. Examples of cancer include, but are not limited to, carcinoma, lymphoma, blastoma, sarcoma and leukemia. More specific examples of such cancers include squamous cell carcinoma, lung cancer (including small cell lung cancer, non-small cell lung cancer, adenocarcinoma of the lung and squamous carcinoma of the lung), peritoneal cancer, hepatocellular cancer, cancer of the stomach or gastric cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial cancer or uterine cancer, salivary gland carcinoma, renal cancer or cancer of the kidney, liver cancer, prostatic cancer, vulval cancer, thyroid cancer, cancer of the liver, and various types of head and neck cancers, as well as B-cell lymphoma (including low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cleaved cell NHL, bulky disease NHL, mantle cell lymphoma, AIDS-related lymphomas, and Waldenstrom macroglobulinemia), chronic lymphocytic leukemia (CLL), acute lymphoblastic leukemia (ALL), hairy cell leukemia, chronic myeloblastic leukemia, post-transplant lymphoproliferative disorder (PTLD), and abnormal vascular proliferation associated with phakomatoses, edema (such as those associated with brain tumors), and Meigs syndrome.

### Antibody

The present invention provides a novel antibody molecule that can be used for the treatment, prevention, and/or diagnosis of various diseases.

The bispecific antibody of the present invention comprises a first antigen-binding site specifically binding to CD3 and a second antigen-binding site specifically binding to CD20. Such antibodies may be referred to herein as, for example, "anti-CD3/anti-CD20", "anti-CD3×CD20", or "CD3×CD20" bispecific molecules, or other similar terms.

The term "anti-CD3 antibody", "anti-CD3", "CD3 antibody", or "CD3-binding antibody" refers to an antibody that is capable of binding to a CD3 protein or a fragment thereof with sufficient affinity such that the antibody can be used as a diagnostic and/or therapeutic agent targeting CD3.

The term "anti-CD20 antibody", "anti-CD20", "CD20 antibody", or "CD20-binding antibody" refers to an antibody that is capable of binding to a CD20 protein or a fragment thereof with sufficient affinity such that the antibody can be used as a diagnostic and/or therapeutic agent targeting CD20.

In some embodiments, the bispecific antibody described herein comprises the following polypeptide chains:
(1) polypeptide chains of formula (I) and formula (II) which bind to domains of CD20:

   VH1-CH1-Fc1 formula (I),

   and

   VL1-CL formula (II);

   and
(2) polypeptide chains of formula (III) and formula (II-2) which bind to domains of CD20 and CD3:

   VH1-CH1-L-VL2-L-VH2-L-Fc2 formula (III),

   and

   VL1-CL formula (II-2);

wherein VH represents a heavy chain variable region; VL represents a light chain variable region; Fc comprises CH2 and CH3; CH1, CH2, and CH3 represent domains 1, 2, and 3 of the heavy chain constant region, respectively; VL represents a light chain variable region; CL represents a light chain constant region; L represents a linker peptide, the linker peptides being identical or different; optionally, a hinge region is present between CH1 and Fc1;
formulas (I), (II), (II-2), and (III) are each formed in a sequential connection from N-terminus to C-terminus;
an antigen-binding site formed by VH1 and VL1 binds to CD20, and an antigen-binding site formed by VL2 and VH2 binds to CD3. In some embodiments, the bispecific antibody TZT6 described herein has the structure described above.

In some embodiments, the bispecific antibody described herein comprises the following polypeptide chains:
(1) polypeptide chains of formula (I) and formula (II) which bind to domains of CD20:

   VH1-CH1-Fc1 formula (I),

   and

   VL1-CL formula (II);

   and
(2) a polypeptide chain of formula (IV) which binds to a domain of CD3:

   VL2-L-VH2-L-Fc2 formula (IV).

wherein VH represents a heavy chain variable region; VL represents a light chain variable region; Fc comprises CH2 and CH3; CH1, CH2, and CH3 represent domains 1, 2, and 3 of the heavy chain constant region, respectively; VL represents a light chain variable region; CL represents a light chain constant region; L represents a linker peptide, the linker peptides being identical or different; optionally, a hinge region is present between CH1 and Fc1;
formulas (I), (II), and (IV) are each formed in a sequential connection from N-terminus to C-terminus;
an antigen-binding site formed by VH1 and VL1 binds to CD20, and an antigen-binding site formed by VL2 and VH2 binds to CD3. In some embodiments, the bispecific antibody TZT7 described herein has the structure described above.

In some embodiments, the linker peptide described herein comprises an amino acid sequence (GGGGS)n, wherein n is each independently selected from 1, 2, 3, 4, 5, or 6; preferably, n is each independently selected from 2, 3, or 4.

In some embodiments, VH1 described herein comprises HCDR1, HCDR2, and HCDR3 having amino acid sequences set forth in SEQ ID NO: 1, SEQ ID NO: 2, and SEQ ID NO: 3, respectively, and VL1 described herein comprises LCDR1, LCDR2, and LCDR3 having amino acid sequences set forth in SEQ ID NO: 4, SEQ ID NO: 5, and SEQ ID NO: 6, respectively.

In some embodiments, provided is the bispecific antibody described herein, wherein:
VH1 comprises an amino acid sequence set forth in SEQ ID NO: 7, or an amino acid sequence having at least 90%, 91%, 92%, 93%, 94%, 95%, 96%, 97%, 98%, or 99% identity to the amino acid sequence set forth in SEQ ID NO: 7; and
VL1 comprises an amino acid sequence set forth in SEQ ID NO: 8; or comprises an amino acid sequence having at least 90%, 91%, 92%, 93%, 94%, 95%, 96%, 97%, 98%, or 99% identity to the amino acid sequence set forth in SEQ ID NO: 8.

In some embodiments, provided is the bispecific antibody described herein, wherein VH2 comprises HCDR1, HCDR2, and HCDR3 having amino acid sequences set forth in SEQ ID NO: 9, SEQ ID NO: 10, and SEQ ID NO: 11, respectively, and VL2 comprises LCDR1, LCDR2, and LCDR3 having amino acid sequences set forth in SEQ ID NO: 12, SEQ ID NO: 13, and SEQ ID NO: 14, respectively.

In some embodiments, provided is the bispecific antibody described herein, wherein:
VH2 comprises an amino acid sequence set forth in SEQ ID NO: 15, or an amino acid sequence having at least 90%, 91%, 92%, 93%, 94%, 95%, 96%, 97%, 98%, or 99% identity to the amino acid sequence set forth in SEQ ID NO: 15; and
VL2 comprises an amino acid sequence set forth in SEQ ID NO: 16, or an amino acid sequence having at least 90%, 91%, 92%, 93%, 94%, 95%, 96%, 97%, 98%, or 99% identity to the amino acid sequence set forth in SEQ ID NO: 16.

In some embodiments, provided is the bispecific antibody described herein, wherein:
the polypeptide chain of formula (I) comprises an amino acid sequence set forth in SEQ ID NO: 17 or 23, or an amino acid sequence having at least 90%, 91%, 92%, 93%, 94%, 95%, 96%, 97%, 98%, or 99% identity to the amino acid sequence set forth in SEQ ID NO: 17 or 23; and
the polypeptide chains of formula (II) and formula (II-2) comprise an amino acid sequence set forth in SEQ ID NO: 18, or an amino acid sequence having at least 90%, 91%, 92%, 93%, 94%, 95%, 96%, 97%, 98%, or 99% identity to the amino acid sequence set forth in SEQ ID NO: 18.

In some embodiments, provided is the bispecific antibody described herein, wherein the polypeptide chain of formula (III) comprises an amino acid sequence set forth in SEQ ID NO: 19, or an amino acid sequence having at least 90%, 91%, 92%, 93%, 94%, 95%, 96%, 97%, 98%, or 99% identity to the amino acid sequence set forth in SEQ ID NO: 19.

In some embodiments, provided is the bispecific antibody described herein, wherein the polypeptide chain of formula (IV) comprises an amino acid sequence set forth in SEQ ID NO: 20, or an amino acid sequence having at least 90%, 91%, 92%, 93%, 94%, 95%, 96%, 97%, 98%, or 99% identity to the amino acid sequence set forth in SEQ ID NO: 20.

In some embodiments, provided is the bispecific antibody described herein, wherein the polypeptide chain of formula (I) comprises an amino acid sequence set forth in SEQ ID NO: 23, the polypeptide chains of formula (II) and formula (II-2) comprise amino acid sequences set forth in SEQ ID NO: 18, and the polypeptide chain of formula (III) comprises an amino acid sequence set forth in SEQ ID NO: 19.

In some embodiments, provided is the bispecific antibody described herein, wherein the polypeptide chain of formula (I) comprises an amino acid sequence set forth in SEQ ID NO: 17, the polypeptide chain of formula (II) comprises an amino acid sequence set forth in SEQ ID NO: 18, and the polypeptide chain of formula (IV) comprises an amino acid sequence set forth in SEQ ID NO: 20.

In some embodiments, the antibody molecule of the present invention is humanized. Different methods for humanizing antibodies are known to those skilled, as reviewed by Almagro & Fransson, the contents of which are incorporated herein in their entireties by reference (Almagro JC and Fransson J (2008) Frontiers in Bioscience 13: 1619-1633).

In some embodiments, the antibody molecule of the present invention is a human or humanized antibody. The human or humanized antibody can be prepared using a variety of techniques known in the art.

In some embodiments, the antibody molecule of the present invention is a chimeric antibody.

In some embodiments, at least a portion of the framework sequences of the antibody molecule disclosed herein is a human consensus framework sequence. In one embodiment, the antibody molecule of the present invention also encompasses antigen-binding fragments thereof, e.g., the following antibody fragments: Fab, Fab', F(ab')₂, Fv, scFv, or sdAb.

In the present invention, multiple anti-human CD3 antibodies are thoroughly analyzed and compared, and subjected to sequence optimization and humanization to obtain the humanized antibody JSCD3.

The humanized anti-CD3 antibody described herein shows stronger tumor-killing activity when used in the preparation of bispecific antibodies. The humanized sequence disclosed herein has more advantages and characteristics than humanized sequences of other companies, shows better anti-tumor activity when used in the preparation of new anti-tumor drugs, has greatly improved physicochemical stability, and is more suitable for the screening and development of anti-tumor drugs. The creative achievement constitutes the beneficial effect and high medical application value of the present invention.

The precise amino acid sequence boundaries of the variable region CDRs of the antibodies of the present invention can be determined using any of many well-known schemes, including Chothia based on the three-dimensional structure of antibodies and the topology of the CDR loops (Chothia et al., (1989) Nature 342: 877-883; Al-Lazikani et al., Standard conformations for the canonical structures of immunoglobulins, Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (1999 Nucleic Acids Research, 27, 209-212), and North CDR definition based on the affinity propagation clustering using a large number of crystal structures.

Unless otherwise stated, the boundaries of the CDRs of the antibodies of the present invention can be determined by those skilled in the art according to any scheme (e.g., different assignment systems or combinations) in the art.

In some embodiments, the amino acid change described herein includes an amino acid deletion, insertion, or substitution. Preferably, the amino acid change described herein is an amino acid substitution, preferably a conservative substitution.

In a preferred embodiment, the amino acid change described herein occurs in a region outside the CDRs (e.g., in FR). More preferably, the amino acid change described herein occurs in a region outside the heavy chain variable region and/or outside the light chain variable region.

In some embodiments, the substitution is a conservative substitution. The conservative substitution refers to the substitution of one amino acid by another amino acid of the same class, e.g., the substitution of an acidic amino acid by another acidic amino acid, the substitution of a basic amino acid by another basic amino acid, or the substitution of a neutral amino acid by another neutral amino acid.

In some embodiments, the bispecific antibodies or the antigen-binding fragments thereof of the present invention include those antibodies having an amino acid sequence that has been mutated by an amino acid deletion, insertion, or substitution but still has at least about 90%, 91%, 92%, 93%, 94%, 95%, 96%, 97%, 98%, 99%, or 100% identity to amino acid sequences of the antibodies described above (particularly in the CDR regions set forth in the above sequences). In some embodiments, when compared to the CDR regions set forth in a particular sequence, the antibodies of the present invention have no more than 1, 2, 3, 4, or 5 amino acid mutations (deletions, insertions, or substitutions) in the CDR regions.

In some embodiments, one or more amino acid modifications may be introduced into an Fc region of the antibody provided herein, thus producing an Fc region variant. The Fc region variant may comprise a human Fc region sequence (e.g., human IgG1, IgG2, IgG3, or IgG4 Fc region) comprising amino acid modifications (e.g., substitutions, deletions, or insertions) at one or more amino acid positions.

In some embodiments, the number of cysteine residues of an antibody can be changed to modify antibody properties. For example, the hinge region of CH1 is modified to change (e.g., increase or decrease) the number of cysteine residues in the hinge region.

The term "Knob into Hole structure" refers to the mutation of the hydrophobic amino acids of CH3 of the antibody Fc. A side-chain amino acid of CH3 of one chain is mutated to form a larger hydrophobic amino acid molecule (knob) to enhance the hydrophobic force. Another side-chain amino acid of CH3 is mutated to form a smaller amino acid (hole) to reduce steric hindrance. After the mutations, the CH3 with Knob and CH3 with Hole form a Knob into Hole structure (KiH) in a hydrophobic manner, which facilitates the formation of a heavy chain heterodimer. The KiH mutation mainly occurs at internal hydrophobic amino acids of the spatial structure of the CH3 domain, and the amino acids exposed to the outside remain virtually unchanged after the mutations, so they do not affect the effector function of Fc and the induced immunogenicity. The term "knob-Fc" refers to the inclusion of a point mutation of T366W in the Fc region of an antibody to form a knob-like spatial structure. Correspondingly, "hole-Fc" refers to the inclusion of point mutations of T366S, L368A, and Y407V in the Fc region of an antibody to form a hole-like spatial structure. Point mutations of S354C and Y349C can further be introduced into knob-Fc and hole-Fc, respectively, to further promote the formation of heterodimers via a disulfide bond. At the same time, point mutations of H435R and Y436F can further be introduced into hole-Fc to reduce the binding to protein A.

The bispecific antibodies of the present invention may comprise two Fc regions, each being part of a separate antibody heavy chain. Fc1 in formula (I) and Fc2 in formula (III) or formula (IV) may have identical sequences, except for mutations in the CH3 domain that are intended to promote or facilitate purification of the heterodimeric (i.e., bispecific) molecule.

In some embodiments, provided is the bispecific antibody described herein, wherein formula (I) and formula (II) are connected via a disulfide bond, and formula (III) and formula (II-2) are connected via a disulfide bond.

In some embodiments, formula (I) and formula (III) of the bispecific antibody may be connected to each other directly or indirectly. In some embodiments, formula (I) and formula (III) of the bispecific antibody may be connected to each other via a linker. In some embodiments, the linker is a peptide linker.

In some embodiments, formula (I) and formula (III) or formula (IV) of the bispecific antibody described herein are connected via a disulfide bond in the Fc region and a Knob into Hole structure of the CH3 domain, preferably, Fc1 in formula (I) is knob-Fc, and Fc2 in formula (III) or formula (IV) is hole-Fc; or Fc1 in formula (I) is hole-Fc, and Fc2 in formula (III) or formula (IV) is knob-Fc.

In some embodiments, provided is the bispecific antibody described herein, wherein CH1-Fc1 in formula (I) and Fc2 in formula (III) are in the form of IgG, e.g., IgG1, IgG2, IgG3, or IgG4, and/or CL in formula (II) and formula (II-2) is derived from a λ or κ chain.

The Fc region of the bispecific antibody of the present invention may be a human Fc region. The Fc region of the bispecific antibody of the present invention may be of any isotype, including, but not limited to, IgG1, IgG2, IgG3, or IgG4. In some embodiments, the Fc regions of the first and second antibodies are both of the IgG1 isotype. In some embodiments, the Fc regions of the first and second antibodies are both of the IgG4 isotype. In some embodiments, one of the Fc regions of the antibody is of the IgG1 isotype and the other is of the IgG4 isotype. In a later embodiment, the resulting bispecific antibody comprises an Fc region of an IgG1 and an Fc region of an IgG4 and thus may have interesting intermediate properties with respect to activation of effector functions.

In some embodiments, the antibodies provided herein can be further modified to contain other non-protein moieties known in the art and readily available. Suitable moieties for antibody derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, glucan, polyvinyl alcohol, polyvinylpyrrolidone, poly-1,3-dioxane, poly-1,3,6-trioxane, ethylene/maleic anhydride copolymer, polyamino acid (homopolymer or random copolymer), and glucan or poly(n-vinylpyrrolidone)polyethylene glycol, propylene glycol homopolymer, polypropylene oxide/ethylene oxide copolymer, polyoxyethylated polyol (such as glycerol), polyvinyl alcohol, and mixtures thereof.

### Antibody Expression

In yet another aspect, the present invention provides a nucleic acid encoding any of the above antibodies or fragments thereof or any one of the chains thereof. In one embodiment, the polynucleotide can include a polynucleotide encoding an amino acid sequence of the light chain variable region and/or heavy chain variable region of the antibody, or a polynucleotide encoding an amino acid sequence of the light chain and/or heavy chain of the antibody.

In some embodiments, the nucleic acid of the present invention includes a nucleic acid encoding an amino acid sequence selected from any one of SEQ ID NOs: 1-20, or a nucleic acid encoding an amino acid sequence having at least 85%, 90%, 91%, 92%, 93%, 94%, 95%, 96%, 97%, 98%, or 99% identity to the amino acid sequence selected from any one of SEQ ID NOs: 1-20.

In yet another aspect, the present invention provides an expression vector comprising the nucleic acid described herein; preferably, the vector is a eukaryotic expression vector. In some embodiments, the nucleic acid described herein is comprised in one or more expression vectors. The vectors include, but are not limited to, viruses, plasmids, cosmids, λ phages, or yeast artificial chromosomes (YACs).

In yet another aspect, the present invention provides a host cell comprising the nucleic acid described herein or the expression vector described herein; preferably, the host cell is a eukaryotic cell; more preferably, the host cell is a mammalian cell (e.g., CHO cell or 293 cell). In another embodiment, the host cell is prokaryotic.

In one embodiment, the present invention provides a method for preparing the anti-CD3×CD20 bispecific antibody, comprising: introducing an expression vector into a mammalian host cell, and culturing the host cell for a period of time sufficient to allow the expression of the antibody in the host cell or more preferably to allow secretion of the antibody into a medium in which the host cell is grown, thereby producing the antibody. The antibody can be isolated from the medium using standard protein purification methods.

The present invention provides a mammalian host cell used for expressing the recombinant antibody of the present invention, which includes a number of immortalized cell lines available from American Type Culture Collection (ATCC). Those cell lines include, in particular, Chinese hamster ovary (CHO) cells, NS0, SP2/0 cells, HeLa cells, baby hamster kidney (BHK) cells, monkey kidney cells (COS), human hepatocellular carcinoma cells, A549 cells, 293T cells, and many other cell lines. Mammalian host cells include human, mouse, rat, dog, monkey, pig, goat, cow, horse, and hamster cells. Particularly preferred cell lines are selected by determining which cell line has a high expression level.

It is likely that antibodies expressed by different cell lines or in transgenic animals have different glycosylations from each other. However, all antibodies encoded by the nucleic acid molecules provided herein or comprising the amino acid sequences provided herein are integral parts of the present invention, regardless of the glycosylation of the antibody. Likewise, in certain embodiments, nonfucosylated antibodies are advantageous because they generally have more potent efficacy *in vitro* and *in vivo* than their fucosylated counterparts, and are unlikely to be immunogenic because their glycan structures are normal components of natural human serum IgG.

### Pharmaceutical Composition and Pharmaceutical Formulation

In yet another aspect, the present invention provides a pharmaceutical composition, which comprises the anti-CD3×CD20 bispecific antibody or the antigen-binding fragment thereof described herein, and a pharmaceutically acceptable carrier or excipient.

It should be understood that the anti-CD3×CD20 antibody or the pharmaceutical composition thereof provided herein can be integrated with a suitable carrier, an excipient and other reagents in a formulation for administration in combination, thus providing improved transfer, delivery, tolerance, etc.

The term "pharmaceutical composition" refers to a formulation that allows the bioactivity of active ingredients comprised therein to be present in an effective form and does not comprise additional ingredients having toxicity unacceptable to a subject to which the formulation is administered.

The pharmaceutical formulation comprising the anti-CD3×CD20 bispecific antibody described herein, preferably in the form of an aqueous solution or a lyophilized formulation, may be prepared by mixing the anti-CD3×CD20 bispecific antibody of the present invention having the desired purity with one or more optional pharmaceutical excipients (Remington's Pharmaceutical Sciences, 16th edition, Osol, A. Ed. (1980)).

The pharmaceutical composition or formulation of the present invention can further comprise one or more additional active ingredients that are required for a specific indication being treated, preferably active ingredients having complementary activities that do not adversely affect one another. In some embodiments, the additional active ingredients are chemotherapeutic agents, immune checkpoint inhibitors, growth inhibitors, antibiotics or various known anti-tumor or anti-cancer agents, which are suitably present in combination in amounts that are effective for the purpose intended.

In some embodiments, the pharmaceutical composition of the present invention further comprises a composition of a nucleic acid encoding the anti-CD3×CD20 bispecific antibody.

In yet another aspect, the present invention provides a drug combination, which comprises the anti-CD3×CD20 bispecific antibody or the antigen-binding fragment thereof described herein, or the pharmaceutical composition described herein, and one or more additional therapeutic agents.

In yet another aspect, the present invention provides a kit, which comprises the antibody or the antigen-binding fragment thereof described herein, the polynucleotide described herein, the expression vector described herein, the host cell described herein, or the pharmaceutical composition described herein.

### Pharmaceutical Use

In yet another aspect, the present invention provides use of the anti-CD3×CD20 bispecific antibody or the antigen-binding fragment thereof described herein, or the pharmaceutical composition described herein, in the preparation of a medicament for preventing and/or treating cancer.

In yet another aspect, the present invention provides the anti-CD3×CD20 bispecific antibody or the antigen-binding fragment thereof described herein, or the pharmaceutical composition described herein, for use in the prevention and/or treatment of cancer.

In yet another aspect, the present invention provides a method for preventing and/or treating cancer, comprising administering to a subject in need thereof the anti-CD3×CD20 bispecific antibody described herein, or the pharmaceutical composition described herein.

In some embodiments, the cancer described herein is selected from acute B-lymphocytic leukemia, diffuse large B-cell lymphoma, chronic lymphocytic leukemia, follicular lymphoma, non-Hodgkin's lymphoma, chronic myelocytic leukemia, or Burkitt's lymphoma.

The subject may be a mammal, e.g., a primate, preferably a higher primate, e.g., a human (e.g., a patient having or at risk of having the disease described herein). In one embodiment, the subject has or is at risk of having the disease described herein (e.g., the tumor described herein). In certain embodiments, the subject receives or has received other therapies, e.g., chemotherapy and/or radiation therapy.

In some embodiments, the routes of administration of the present invention include, but are not limited to, oral administration, intravenous administration, subcutaneous administration, intramuscular administration, intra-arterial administration, intra-articular administration (e.g., in arthritic joints), administration by inhalation or aerosol delivery, intratumoral administration, and the like.

In some embodiments, the present invention provides co-administering to a subject a therapeutically effective amount of one or more therapies (e.g., therapeutic modalities and/or additional therapeutic agents).

In some embodiments, the method or use provided herein also comprises administering to the individual one or more therapies (e.g., therapeutic modalities and/or additional therapeutic agents). The antibody of the present invention can be used alone or used in combination with additional therapeutic agents in a therapy. For example, the antibody may be co-administered with at least one additional therapeutic agent.

In some embodiments, the therapeutic modality described above comprises surgery; radiation therapy, partial irradiation, focused irradiation, or the like. In some embodiments, the therapeutic agent described above is selected from a chemotherapeutic agent, a cytotoxic agent, a vaccine, another antibody, an anti-infective active agent, or an immunomodulatory agent.

### Methods for Diagnosis and Detection

In yet another aspect, the present invention provides a method for detecting the presence of CD3 or CD20 in a sample using any of the antibodies or the antigen-binding fragments thereof described herein. The term "detection" as used herein includes quantitative or qualitative detection. Exemplary detection methods may involve immunohistochemistry, immunocytochemistry, flow cytometry (e.g., FACS), magnetic beads complexed with antibody molecules, ELISA assays, and PCR-techniques (e.g., RT-PCR). In some embodiments, the sample is a biological sample. In some embodiments, the biological sample is blood, serum, or other liquid samples of biological origin. In certain embodiments, the biological sample includes cells or tissues. In some embodiments, the biological sample is from a hyperproliferative or cancerous lesion.

In one aspect, the present invention provides a diagnostic method for detecting the presence of a CD3 or CD20 antigen in a biological sample, e.g., serum, semen, or urine or tissue biopsy (e.g., from a hyperproliferative or cancerous lesion), *in vitro* or *in vivo.* The diagnostic method comprises: (1) making a sample (and optionally a control sample) be contact with the antibody molecule described herein or administering to a subject the antibody molecule under conditions that allow an interaction to occur, and (2) detecting the formation of a complex between the antibody molecule and the sample (and optionally the control sample). The formation of a complex indicates the presence of the antigen of interest and may indicate the applicability or need for treatment and/or prevention described herein. In some embodiments, the present invention provides a diagnostic kit comprising the antibody molecule or the antigen-binding fragment thereof described herein, or the pharmaceutical composition described herein, and instructions for use.

The present invention includes any combinations of the specific embodiments described. Further embodiments of the present invention and the full scope of applicability will become apparent from the detailed description provided below. However, it should be understood that the detailed description and the specific examples, while indicating preferred embodiments of the present invention, are provided by way of illustration only, as various changes and modifications within the spirit and scope of the present invention will become apparent to those skilled in the art from the detailed description. All publications, patents and patent applications cited herein, including the citations, are hereby incorporated by reference in their entireties for all purposes.

The following abbreviations are used in the present invention:
His-tag for a histidine tag;
Fc tag for a crystallizable fragment tag;
ECD for an extracellular domain;
PEI for polyethyleneimine;
BSA for bovine serum albumin;
PBS for phosphate buffered saline;
FBS for fetal bovine serum;
CFSE for carboxyfluorescein diacetate succinimidyl ester;
APC for allophycocyanin;
NA-PE for phycoerythrin-labeled neutral avidin;
PE for phycoerythrin;
TMB for 3,3',5,5'-tetramethylbenzidine; and
PBST for phosphate buffered saline with tween.

### Examples

The present invention is illustrated by the following examples, which, however, are not intended to be limiting in any way. The present invention has been described in detail, and the specific embodiments are also disclosed. It will be apparent to those skilled in the art that various changes and modifications can be made to the specific embodiments of the present invention without departing from the spirit and scope of the present invention.

### Example 1. Construction of Expression Vectors for Bispecific Antibody Molecules 1.1 Anti-CD3 antibody

In the present invention, multiple anti-human CD3 antibodies were analyzed, among which one murine antibody SP34 was subjected to sequence optimization and humanization to obtain a humanized anti-CD3 antibody JSCD3.

SP34 is a murine anti-CD3 monoclonal antibody, which comprises a heavy chain having an amino acid sequence of SEQ ID NO: 24, and a light chain having an amino acid sequence of SEQ ID NO: 25.

The amino acid sequences of the humanized anti-CD3 antibody JSCD3 are as follows:
JSCD3 VH: SEQ ID NO: 15, with amino acid sequences of heavy chain complementarity determining regions HCDR1, HCDR2, and HCDR3 set forth in SEQ ID NOs: 9, 10, and 11, respectively; and
JSCD3 VL: SEQ ID NO: 16, with amino acid sequences of light chain complementarity determining regions LCDR1, LCDR2, and LCDR3 set forth in SEQ ID NO: 12, 13, and 14, respectively.

The full-length amino acid sequences of JSCD3 are as follows:
JSCD3 HC: SEQ ID NO: 21
JSCD3 LC: SEQ ID NO: 22

### 1.2 Construction of expression vectors for bispecific antibody molecules Bispecific antibody molecule TZT6:

It was obtained by co-transient expression through HXT2-JS CD20 LC-2, HXT4s-JS CD20 HC-2 b, and HX4-JSCD3ScFv NVL-Mut. The expression vectors were sequentially constructed as follows: the gene encoding JSCD20 LC-2 was synthesized by entrusted Genscript Biotech Ltd., digested with BSPQI, and ligated to the HXT2 vector (a vector engineered in-house by Junshi Biosciences, derived from pCDNA3.1) to obtain the first expression vector HXT2-JS CD20 LC-2. The gene encoding JSCD20 HC-2 was synthesized by entrusted Genscript Biotech Ltd., digested with HindIII and NheI, and ligated to HXT4s-Mut-b (a vector engineered in-house by Junshi Biosciences, derived from pCDNA3.1) to obtain the second expression vector HXT4s-JS CD20 HC-2 b. The gene encoding JSCD3ScFv was synthesized by entrusted Genscript Biotech Ltd., digested with HindIII and NheI, and ligated to HX4-FC-mut-h (a vector engineered in-house by Junshi Biosciences, derived from pCDNA3.1) to obtain the third expression vector HX4-JSCD3ScFv NVL-Mut h.

### Bispecific antibody molecule TZT7:

It was obtained by co-transient expression through HXT2-JS CD20 LC-2, HXT4s-JS CD20HC-2 Mut h, and HX4-JS CD20 HC-2 JS CD3ScFv NVL-G4 FC b V2. The expression vectors were sequentially constructed as follows: the gene encoding JSCD20 LC-2 was synthesized by entrusted Genscript Biotech Ltd., digested with BSPQI, and ligated to the HXT2 vector to obtain the first expression vector HXT2-JS CD20 LC-2. The gene encoding JSCD20 HC-2 was synthesized by entrusted Genscript Biotech Ltd., digested with HindIII and NheI, and ligated to HXT4s-Mut-h to obtain the second expression vector HXT4s-JS CD20 HC-2 h. The gene encoding JSCD3ScFv was synthesized by entrusted Genscript Biotech Ltd., digested with HindIII and NheI, and ligated to the HX4-JSCD20HC-2-G4 FC mut b vector to obtain the third expression vector HX4-JS CD20 HC-2 JS CD3ScFv NVL-G4 FC b V2.

### Example 2. Expression and Purification of Bispecific Antibody Molecules

### 2.1. Expression of bispecific antibody molecules

Transient expression and purification of bispecific antibody molecules TZT6 and TZT7: 3 plasmids constructed above were extracted in large quantities with an endotoxin-controlling kit before subsequent use for expression by mammalian cells. CHO-K1 cells (engineered at the genomic level so that the cells were suitable for transient expression) being cultured were counted and, when the cell density was 2-6 × 10⁶/mL, expanded by subculturing in a CD CHO medium. The cells were diluted to a density of 1.5-2.0 × 10⁶/mL the day before transfection. The next day, transfection was performed when the cell density reached about 3.5 × 10⁶/mL. One tenth of transfection volume of medium was added first, then 1-2 µg/mL transfection volume of plasmid, and finally 3-14 µg/mL of PEI. They were well mixed and then incubated at room temperature for no more than 30 min. Finally, the transfection mixture was slowly added to the pre-treated cells, and they were well mixed as the mixture was added. The mixture after the transfection was cultured on a shaker at 36.5 °C at 120 rmp with 7% CO₂. The culture was performed for 6-10 days after the transfection, and the medium supplementation was performed once every two days.

### 2.2. Purification of bispecific antibody molecules

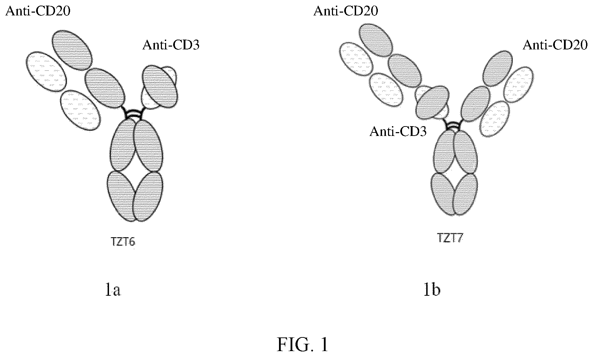

After the culture of the above transfection mixture was completed, the precipitate was discarded by centrifugation at 1000 g for 10 min, and then cell supernatant was collected by centrifugation at 12000 g for 30 min and sterile-filtered. Purification was performed on an AKTA Avant purifier. A column packed with the Praesto Jetted A50 affinity packing was first subjected to CIP with 0.1 M NaOH for 15-20 min and then equilibrated with 3-5 CVs of PBS before sample loading. After the sample loading was completed, the column was rinsed with an affinity chromatography elution buffer. Finally, the target protein was eluted with a pH 3.8 acetic acid-sodium acetate buffer. The sample was neutralized and adjusted to pH 5.5 with a 1 M Tris buffer. Part of the sample was sent for SEC-HPLC analysis to determine the purity of the sample. The remaining sample was subjected to polishing purification. The weak cationic packing EMD COOM was selected for the polishing purification. The equilibration buffer was a pH 5.5, 50 mM acetic acid-sodium acetate system. The eluent was a pH 5.5, 50 mM acetic acid-sodium acetate + 1 M NaCl buffer system. Linear elution was adopted to collect the target protein. The final SEC-HPLC monomer purity could reach 95% or above. The bispecific antibody molecules TZT6 and TZT7 were obtained. The schematic structural diagram of the bispecific antibody molecule TZT6 is shown in FIG. 1a, and the schematic structural diagram of the bispecific antibody molecule TZT7 is shown in FIG. 1b.

The amino acid sequences of the bispecific antibody molecule TZT6 are shown as follows:
HC1 (HXT4s-JS CD20 HC-2 b): SEQ ID NO: 17
LC (HXT2-JS CD20 LC-2): SEQ ID NO: 18
HC2 (HX4-JSCD3ScFv NVL-Mut h): SEQ ID NO: 20

The amino acid sequences of the bispecific antibody molecule TZT7 are shown as follows:
HC1 (HXT4s-JS CD20HC-2 Mut h): SEQ ID NO: 23
LC (HXT2-JS CD20 LC-2): SEQ ID NO: 18
HC2 (HX4-JS CD20 HC-2 JS CD3ScFv NVL-G4 FC b V2): SEQ ID NO: 19.

The amino acid sequences of CDRs and variable regions of the bispecific antibody molecules TZT6 and TZT7 are shown in Table 1.

**Table 1: Amino acid sequences of CDRs and variable regions of bispecific antibody molecules (KABAT scheme)**

| | | HCDR1 | HCDR2 | HCDR3 | LCDR1 | LCDR2 | LCDR3 | VH | VL |
|---|---|---|---|---|---|---|---|---|---|
| TZT6 | HC1/LC | SEQ ID NO:1 | SEQ ID NO:2 | SEQ ID NO:3 | SEQ ID NO:4 | SEQ ID NO:5 | SEQ ID NO:6 | SEQ ID NO:7 | SEQ ID NO:8 |
| | HC2 | SEQ ID NO:9 | SEQ ID NO: 10 | SEQ ID NO: 11 | SEQ ID NO: 12 | SEQ ID NO: 13 | SEQ ID NO: 14 | SEQ ID NO:15 | SEQ ID NO:16 |
| TZT7 | HC1/LC | SEQ ID NO:1 | SEQ ID NO:2 | SEQ ID NO:3 | SEQ ID NO:4 | SEQ ID NO:5 | SEQ ID NO:6 | SEQ ID NO:7 | SEQ ID NO:8 |
| | HC2/LC(CD20) | SEQ ID NO:1 | SEQ ID NO:2 | SEQ ID NO:3 | SEQ ID NO:4 | SEQ ID NO:5 | SEQ ID NO:6 | SEQ ID NO:7 | SEQ ID NO:8 |
| | HC2(CD3) | SEQ ID NO:9 | SEQ ID NO: 10 | SEQ ID NO: 11 | SEQ ID NO: 12 | SEQ ID NO: 13 | SEQ ID NO: 14 | SEQ ID NO:15 | SEQ ID NO:16 |

### Example 3. Stability of Bispecific Antibodies

### 3.1 Thermostability of bispecific antibodies

### 1. Objective

To test the thermostability of the bispecific antibodies of the present invention. The stability of the bispecific antibodies in a conventional formulation (20 mM citric acid-sodium citrate buffer, 50 mM sodium chloride, 140 mM mannitol, pH 6.0) was investigated by DSF (differential scanning fluorescence technique).

### 2. Procedures and results

The antibody samples were each added to the above buffer, with the concentration of the antibody samples controlled to about 4 mg/mL, and DSF tests were performed. As shown in Table 2, the results showed that the bispecific antibodies TZT6 and TZT7 both had a melting temperature (Tm) of 60 °C or higher in the above buffer, indicating good thermostability.

**Table 2: Thermostability tests for bispecific antibodies**

| Sample | Tm1 (°C) | Tm2 (°C) |
|---|---|---|
| TZT6 | 62.3 | 72.7 |
| TZT7 | 62.7 | 75.1 |

### 3.2. High-temperature stability of bispecific antibodies

### 1. Objective

To test the stability of the bispecific antibodies at high temperature.

### 2. Procedures and results

The antibody samples were each added to a pH 6.0 buffer system (20 mM citric acid-sodium citrate/50 mM sodium chloride/140 mM mannitol), with the concentration of the samples controlled at about 4 mg/mL, and the resulting mixture was aliquoted into vials at 500 µL/vial. The vials were placed in an incubator at 40 °C to investigate stability at 0 W, 2 W, and 4 W. The samples were sent for analysis according to Table 3. Stability was evaluated by the following parameters: (a) the content of antibody monomers, aggregates, or fragments determined by SEC-HPLC (size exclusion chromatography); (b) the molecular weight of the antibody determined by two CE-SDS (sodium dodecyl sulfate capillary electrophoresis) methods; and (c) the bioactivity of the antibody determined by ELISA and reporter gene methods (see Example 4 and Example 8 for experimental procedures).

The assay results showed that after storage at a high temperature of 40 °C for 4 weeks, the bispecific antibodies TZT6 and TZT7 of the present invention showed no significant changes in the purity (SEC-HPLC, R-CE-SDS (reduction electrophoresis), and NR-CE-SDS (non-reduction electrophoresis)) and the bioactivity, indicating good thermostability. The specific results are shown in Table 3.

**Table 3: Stability of bispecific antibodies during storage at a high temperature of 40 °C for 4 weeks**

| Sample | Time | SEC-HPLC | | | NR-CE-SDS | R-CE-SDS | Bioactivity | |
|---|---|---|---|---|---|---|---|---|
| | | Aggregate | Monomer | Fragment | | | Relative binding activity Elisa (%) | Relative cell viability |
| | | | | | | | | Reporter gene method (%) |
| TZT6 | 0 week | 1.2% | 97.6% | 1.3% | 96.2% | 99.3% | 119.4 | 91 |
| | 2 weeks | 1.8% | 96.4% | 1.9% | 96.1% | 99.3% | 100.2 | 112 |
| | 4 weeks | 2.2% | 96.0% | 1.8% | 96.1% | 99.2% | 104.3 | 105 |
| TZT7 | 0 week | 1.3% | 98.6% | 0.1% | 99.7% | 98.7% | 101.9 | 90 |
| | 2 weeks | 1.6% | 97.1% | 1.3% | 98.7% | 98.2% | 99.7 | 106 |
| | 4 weeks | 2.2% | 96.1% | 1.7% | 98.7% | 98.0% | 102.1 | 78 |

### Example 4. Assay on Binding of Bispecific Antibodies to Human CD3ε by ELISA

### 1. Objective

To assay the binding of the bispecific antibodies of the present invention to human CD3ε by an ELISA method.

### 2. Procedures

a. A 96-well plate was coated at 100 µL/well with recombinant human CD3ε (Novoprotein, C578) at a concentration of 1.0 µg/mL as an antigen, and incubated at 37 °C for 1.5 h.
b. The plate was washed 4 times with 1× PBST at 300 µL/well, and 2% BSA was added at 200 µL/well. The plate was blocked at 37 °C for 1.5 h.
c. The plate was washed 4 times with 1× PBST at 300 µL/well, and the bispecific antibodies TZT6 and TZT7 and a negative control antibody (anti-KLH hIgG4) subjected to gradient dilution (starting at 10 µg/mL, 2.5-fold gradient dilution for 12 concentration points, 100 µL/well) were added. The plate was incubated at 37 °C for 1 h.
d. The plate was washed 4 times with 1× PBST at 300 µL/well.
e. 5000-fold diluted horseradish peroxidase (HRP)-conjugated mouse anti-human IgG4 Fc antibody (Southern Biotech, 9200-05) was added at 100 µL/well, and the plate was incubated at 37 °C for 1 h.
f. The plate was washed 4 times with 1× PBST at 300 µL/well.
g. 0.1 mg/mL TMB was added at 100 µL/well, and after the plate was incubated at 37 °C for 15 min, a 2 M hydrochloric acid solution was added at 100 µL/well to stop the reactions.
h. The absorbance was measured at 450 nm/620 nm on a microplate reader, and data were analyzed using Graphpad Prism 7.

### 3. Results

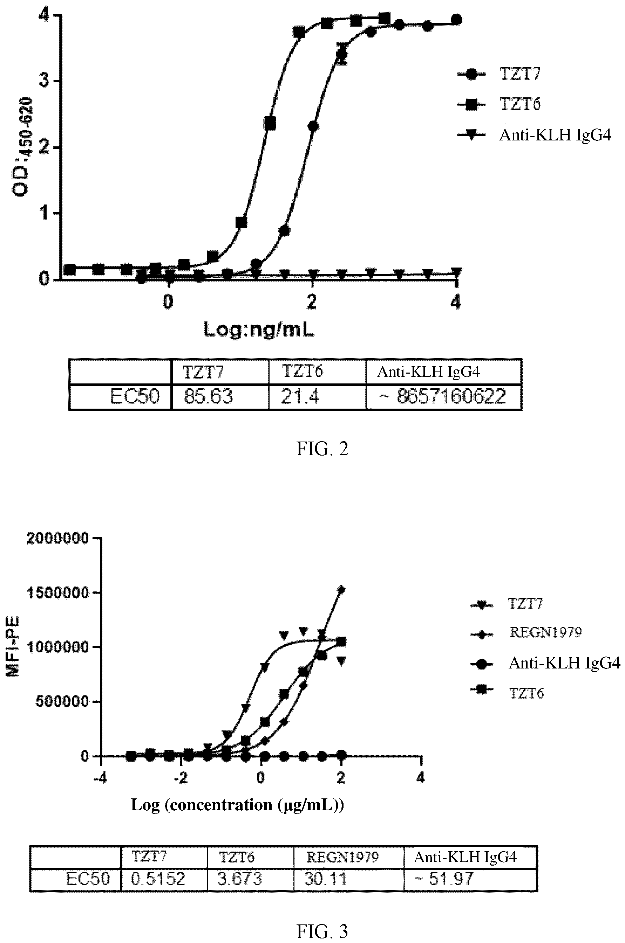

As shown in FIG. 2, the bispecific antibodies TZT6 and TZT7 of the present invention had strong binding to human CD3ε, with EC₅₀ values being 21.4 ng/mL and 85.63 ng/mL, respectively.

### Example 5. Binding of Bispecific Antibodies to Raji Cells

Raji cells (human B lymphoma cells, CD20 positive) were incubated with different concentrations of the bispecific antibodies TZT7 and TZT6 (starting at 100 µg/mL, 3-fold dilution, 12 concentration gradients in total) at 4 °C for 30 min, then washed and incubated with a fluorescence-labeled secondary antibody at 4 °C for 30 min in the dark. Finally, the cells were collected by using a flow cytometer (BD Canto II), and the fluorescent antibodies bound to the cell surface were detected. Raw data were analyzed by using FlowJo to obtain MFI values, the antibody dose-dependent binding curves were fitted by using GraphPad (FIG. 3), and EC₅₀ was calculated. The positive control and the negative control were REGN1979 (Regeneron Pharmaceuticals) and anti-KLH hu-IgG4 antibody, respectively.

As shown in FIG. 3, TZT7, TZT6, and REGN1979 were all able to bind with high affinity to CD20 on the surface of Raji cells, with EC₅₀ values being 0.5152 µg/mL, 3.673 µg/mL, and 30.11 µg/mL, respectively, and the binding ability of TZT7 and TZT6 to Raji cells was significantly better than that of the positive control REGN1979.

### Example 6. Binding of Bispecific Antibodies to Jurkat Cells

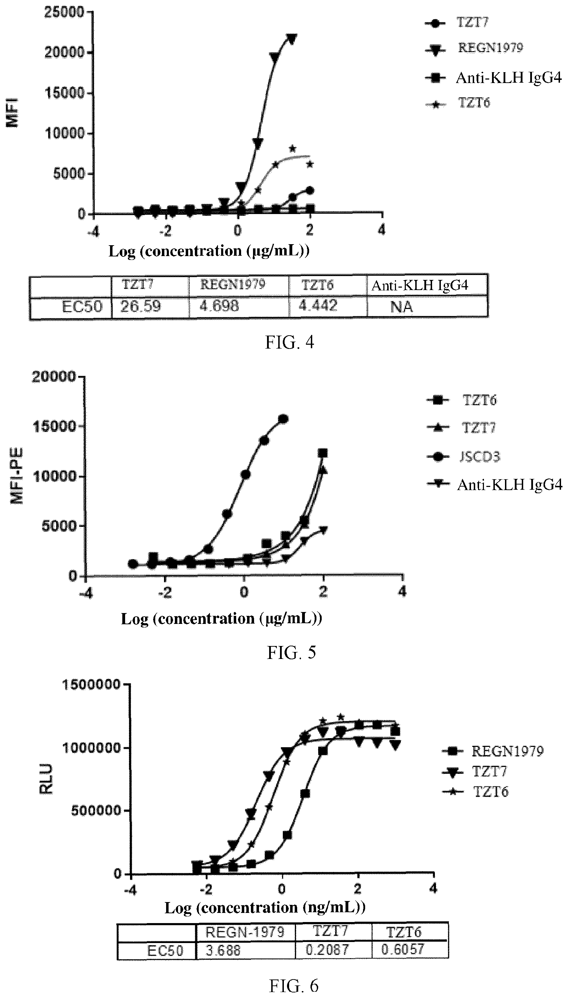

Jurkat cells (human T lymphoma cells, CD3-positive) were incubated with different concentrations of the bispecific antibodies TZT6 and TZT7 (starting at 100 µg/mL, 3-fold dilution, 12 concentration gradients in total) at 4 °C for 30 min, then washed and incubated with a fluorescence-labeled secondary antibody at 4 °C for 30 min in the dark. Finally, the cells were collected by using a flow cytometer (BD Canto II), and the fluorescent antibodies bound to the cell surface were detected. Raw data were analyzed by using FlowJo to obtain MFI values, the antibody dose-dependent binding curves were fitted by using GraphPad (FIG. 4), and EC₅₀ was calculated. The positive control and the negative control were REGN1979 (Regeneron Pharmaceuticals) and anti-KLH hu-IgG4 antibody, respectively.

As shown in FIG. 4, REGN1979, TZT6, and TZT7 were all able to bind to human CD3 on the surface of Jurkat cells, with EC₅₀ values being 4.698 µg/mL, 4.442 µg/mL, and 26.59 µg/mL, respectively.

### Example 7. Binding of Bispecific Antibodies to Cells Overexpressing Cynomolgus Monkey CD3e

CHO Cyno CD3e cells (overexpressing cynomolgus monkey CD3e on the surface of CHO cells) were incubated with different concentrations of the bispecific antibodies TZT7 and TZT6 (starting at 100 µg/mL, 3-fold dilution, 12 concentration gradients in total) at 4 °C for 30 min, then washed and incubated with a fluorescence-labeled secondary antibody at 4 °C for 30 min in the dark. Finally, the cells were collected by using a flow cytometer (BD Canto II), and the fluorescent antibodies bound to the cell surface were detected. Raw data were analyzed by using FlowJo to obtain MFI values, the antibody dose-dependent binding curves were fitted by using GraphPad (FIG. 5), and EC₅₀ was calculated. The positive control and the negative control were JSCD3 (anti-CD3 antibody) and anti-KLH hu-IgG4 antibody, respectively.

As shown in FIG. 5, the bispecific antibodies TZT7 and TZT6 were both able to bind to cynomolgus monkey CD3e.

### Example 8. Activity of Bispecific Antibodies by Luciferase Reporter Assay

Target cells Raji (human B lymphoma cells, CD20 positive) and effector cells Jurkat NFAT (stably expressing luc2P/NFAT-RE) were added to a 96-well flat-bottomed white plate (Corning, Cat # 3917) at 5 × 10⁴ cells/well and 1 × 10⁵ cells/well, respectively. Then, the bispecific antibodies TZT7, TZT6, and REGN1979 (starting at 1 µg/mL, 3-fold dilution, 12 concentration gradients in total) were added to the cell plate with an experimental buffer (RPMI 1640 (1×) + 2% FBS). The plate was incubated in an incubator at 37 °C for 4-6 h. Finally, a ONE-Glo luciferase assay reagent (Promega) was added to the cell-antibody mixed system, and chemiluminescence signals were detected by using a multi-mode microplate reader (TECAN M1000 pro). A four-parameter regression curve was fitted by using GraphPad prism software and EC₅₀ values were calculated.

As shown in FIG. 6, TZT7, TZT6, and REGN1979 (Regeneron Pharmaceuticals) had strong T cell activation activity by a luciferase reporter assay consisting of effector cells Jurkat NFAT and target cells Raji, with EC₅₀ values being 0.2087 ng/mL, 0.6057 ng/mL, and 3.688 ng/mL respectively, and the T cell activation activity of TZT7 and TZT6 was significantly better than that of REGN1979.

### Example 9. Assay on Activation Activity of Bispecific Antibodies for T lymphocytes

The anti-CD3/CD20 bispecific antibodies can effectively promote the activation of T lymphocytes in peripheral blood mononuclear cells. Generally, the up-regulation of the expression of a cell surface marker CD69 is a marker of the early stage of T cell activation, and the up-regulation of the expression of a cell surface marker CD25 is a marker of the later stage of T cell activation. In this study, the up-regulation of the proportion of CD25 and CD69 double-positive populations was used for evaluating the activation effect of the anti-CD3/CD20 bispecific antibodies on the T cells.

Human pan T cells were isolated and purified from commercial PBMCs (Allcells, Cat # PB004F-C) using a human pan T cell isolation kit (Miltenyi Biotec, Cat # 130-096-535). The purified human pan T cells (1 × 10⁵ cells/well) and Raji cells (2 × 10⁴ cells/well) were then co-incubated with TZT7 and TZT6 or a control sample subjected to gradient dilution (starting at 10 µg/mL, 5-fold dilution, 12 concentration gradients in total) in a 96-well plate at 37 °C for 24 h. Finally, the cells were collected, stained with APC anti-human CD8a (Biolegend, Cat # 301049), PE anti-human CD25 (Biolegend, Cat# 302606), and BV421 anti-human CD69 (BD, Cat # 562884), and then assayed on a flow cytometer (BD, CantoII). The proportion of CD25⁺CD69⁺ double-positive cell populations on CD8⁺ T cells was derived using FlowJo software, a four-parameter regression curve was fitted by using GraphPad prism software, and EC₅₀ values were calculated.

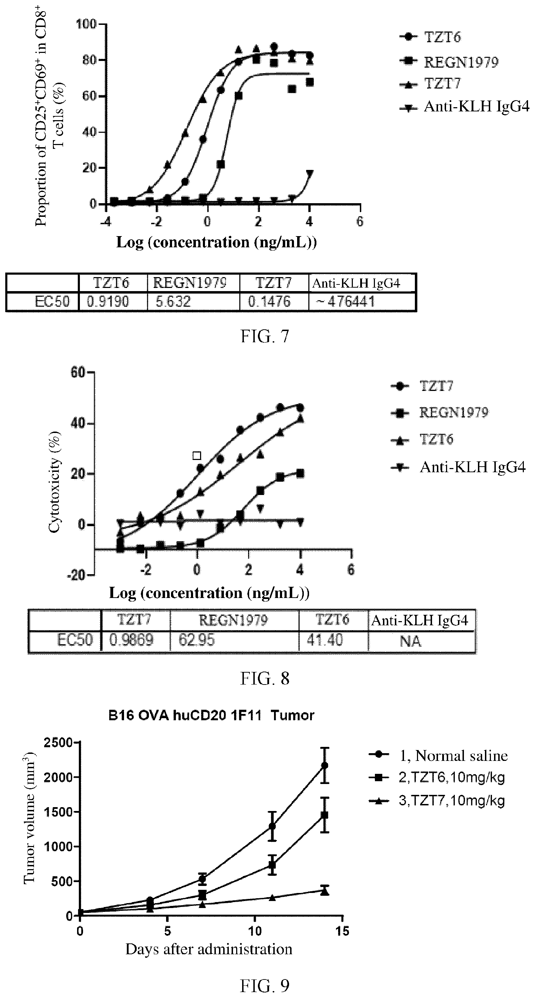

As shown in FIG. 7, the anti-CD3/CD20 bispecific antibodies TZT7 and TZT6 of the present invention and the positive control REGN1979 (Regeneron Pharmaceuticals) were all able to effectively promote an increase in the proportion of CD25⁺ and CD69⁺ double-positive cell populations in CD8 T lymphocytes, indicating the promotion of T cell activation, with EC₅₀ values being 0.1476 ng/mL, 0.9190 ng/mL, and 5.632 ng/mL, respectively. The T cell activation activity of TZT7 and TZT6 was significantly better than that of REGN1979.

### Example 10. Assay on Activity of Bispecific Antibodies for Promoting Killing of B Lymphoma Cells by T Cells

Flow cytometry was used to determine the ability of the anti-CD3/CD20 bispecific antibodies of the present invention to promote the killing of B lymphoma cells (Raji cells) by T cells.

Human pan T cells were first isolated and purified from commercial PBMCs (Allcells, Cat # PB004F-C) using a human pan T cell isolation kit (Miltenyi Biotec, Cat # 130-096-535). The Raji cells were then labeled with a CFSE cell proliferation kit (Invitrogen, Cat # C34554) and the purified human pan T cells (1 × 10⁵ cells/well) and CFSE-labeled Raji cells (2 × 10⁴ cells/well) were co-incubated with TZT7, TZT6, or a control sample subjected to gradient dilution (starting at 10 µg/mL, 6-fold dilution, 10 concentration gradients in total) in a 96-well plate at 37 °C for 72 h. Finally, the cells were collected, stained with 7-AAD (BD, Cat # 559925), and then collected by flow cytometry (BD, CantoII). The proportion of killed Raji cell populations, i.e., the proportion of CFSE⁺7-AAD⁺ (Percp-cy5.5 channel) double-positive populations, was analyzed by using FlowJo software, a four-parameters regression curve was fitted by using GraphPad prism software, and EC₅₀ values were calculated.

As shown in FIG. 8, the anti-CD3/CD20 bispecific antibodies TZT7 and TZT6 of the present invention and REGN1979 (Regeneron Pharmaceuticals) were all able to effectively promote the killing of B lymphoma cells (i.e., Raji cells) by T cells, with EC₅₀ values being 0.9869 ng/mL, 41.40 ng/mL, and 62.95 ng/mL, respectively. The killing activity of TZT7 was significantly better than that of REGN1979.

### Example 11. Assay on Single-Dose PK in Cynomolgus Monkeys

### 1. Objective

To investigate the single-dose PK of the bispecific antibodies of the present invention in cynomolgus monkeys.

### 2. Procedures

a. A 96-well plate was coated at 100 µL/well with recombinant human CD3ε (Novoprotein, C578) at a concentration of 1.0 µg/mL as an antigen, and incubated at 37 °C for 1.5 h.
b. The plate was washed 4 times with 1× PBST at 300 µL/well, and 2% BSA was added at 200 µL/well. The plate was blocked at 37 °C for 1.5 h.
c. A TZT7 standard curve sample was prepared by using blank cynomolgus monkey serum, with a range of 8µg/mL-125 ng/mL; a TZT6 standard curve sample was prepared by using blank cynomolgus monkey serum, with a range of 4µg/mL -62.5 ng/mL; and the test sample was diluted to a standard curve range by using blank cynomolgus monkey serum. Then, the standard curve samples and the test sample were each subjected to a 10-fold dilution with 2% BSA. The test sample prepared above was added at 100 µL/well, and the plate was incubated at 37 °C for 1 h and then washed.
d. The plate was washed 4 times with 1× PBST at 300 µL/well.
e. 5000-fold diluted horseradish peroxidase (HRP)-conjugated mouse anti-human IgG4 Fc antibody (Southern Biotech, 9200-05) was added at 100 µL/well, and the plate was incubated at 37 °C for 1 h.
f. The plate was washed 4 times with 1× PBST at 300 µL/well.
g. 0.1 mg/mL TMB was added at 100 µL/well, and after the plate was incubated at 37 °C for 5-7 min, a 2 M hydrochloric acid solution was added at 100 µL/well to stop the reactions.
h. The absorbance was measured at 450 nm/620 nm on a microplate reader, and data were analyzed using a four-parameter model of SoftMax Pro5.4.1.

### 3. Results

As shown in Table 4, when the *in vivo* administration dose of the bispecific antibody TZT7 in cynomolgus monkeys was 1 mg/kg, the half-life was 19.1 h, and the Cₘₐₓ was 24.1 mg/L; when the administration dose was 10 mg/kg, the half-life was 138.3 h, and the Cₘₐₓ was 348.2 mg/L. When the *in vivo* administration dose of the bispecific antibody TZT6 in cynomolgus monkeys was 1 mg/kg, the half-life was 164.2 h, and the Cₘₐₓ was 24.1 mg/L; when the administration dose was 10 mg/kg, the half-life was 168.8 h, and the Cₘₐₓ was 244.9 mg/L.

**Table 4: Results for single-dose PK in cynomolgus monkeys**

| PK parameters | TZT7 | | TZT6 | |
|---|---|---|---|---|
| | Administration dose 1 mg/kg Average ± SD | Administration dose 10 mg/kg Average ± SD | Administration dose 1 mg/kg Average ± SD | Administration dose 10 mg/kg Average ± SD |
| AUC₍₀₋ₜ₎/mg/L*h | 402.2±17.5 | 8122.2±1019.3 | 1240.7±11.9 | 17250.2±6826.7 |
| t_{1/2}/h | 19.1±2.0 | 138.3±21.0 | 164.2±54.0 | 168.8±87.4 |
| Tₘₐₓ/h | 0.5±0.0 | 1.25±1.1 | 0.5±0.0 | 0.5±0.0 |
| Cₘₐₓ/mg/L | 24.1±2.5 | 348.2±8.1 | 24.1±1.1 | 244.9±75.7 |

| | | | | |
|---|---|---|---|---|
| Note: AUC₍₀₋ₜ₎: area under the drug concentration-time curve; t_{1,2}: terminal elimination half-life; Tₘₐₓ: time to peak; Cₘₐₓ: peak concentration of drug. | | | | |

### Example 12. Inhibitory Effect of Bispecific Antibodies on Growth of B16 OVA huCD20 Tumor

### 1. Objective

To evaluate the anti-tumor effect of TZT6 and TZT7 of the present invention in a B16 OVA huCD20 subcutaneous xenograft tumor model of murine melanoma.

### 2. Procedures

Female hCD3e humanized mice (purchased from Biocytogen Jiangsu Co., Ltd.) at 6-8 weeks of old were inoculated subcutaneously on the right dorsal side with 2.5 × 10⁵ B16 OVA huCD20 1F11 cells (transgenic human CD20 gene). When the mean tumor volume was about 52 mm³, appropriate animals were selected and randomly divided into 3 groups of 6 animals each based on tumor volume, which were
a solvent control group:
   - G1 normal saline group; and
treatment groups:
   - G2 TZT6 (10 mg/kg) group, and
   - G3 TZT7 (10 mg/kg) group.

All groups were subjected to intraperitoneal injection twice a week for 2 consecutive weeks, and the experiment ended 4 days after the last administration. Tumor volume and body weight of mice were measured and recorded twice a week. At the end of the experiment, mice were euthanized and the tumor inhibition rate was calculated: TGI (%) = [1 - (Ti - T0)/(Vi - V0)] × 100%. (Ti: the mean tumor volume of the treatment group on day i of administration, T0: the mean tumor volume of the treatment group on day 0 of administration; Vi: the mean tumor volume of the solvent control group on day i of administration, V0: the mean tumor volume of the solvent control group on day 0 of administration).

As shown in FIG. 9, on day 14 after the administration, the mean tumor volume of the normal saline control group was 2170 mm³, the mean tumor volume of the TZT6 group was 1455 mm³, and the tumor inhibition rate was 33.7% as compared to the normal saline control group. The mean tumor volume of the TZT7 group was 372 mm³, and the tumor inhibition rate was 84.9% as compared to the normal saline control group, indicating that the tumor volume increase was significantly inhibited. The results showed that TZT6 and TZT7 both had a tumor inhibitory effect at a dose level of 10 mg/kg in the B16 OVA huCD20 1F11 xenograft tumor model of hCD3e humanized mice.

### Example 13. Inhibitory Effect of TZT7 on Growth of B16 OVA huCD20 Tumor

### 1. Objective

To evaluate the anti-tumor effect of TZT7 of the present invention in a B16 OVA huCD20 subcutaneous xenograft tumor model of murine melanoma.

### 2. Procedures

Female hCD3e humanized mice (purchased from Biocytogen Jiangsu Co., Ltd.) at 6-8 weeks of old were inoculated subcutaneously on the right dorsal side with 2.5 × 10⁵ B16 OVA huCD20 1F11 cells (transgenic human CD20 gene). When the mean tumor volume was about 88 mm³, appropriate animals were selected and randomly divided into 3 groups of 6 animals each based on tumor volume, which were
a solvent control group:
   - G1 normal saline group; and
treatment groups:
   - G2 TZT7 (10 mg/kg) group, and
   - G3 RENG1979 (10 mg/kg) group.

All groups were subjected to intraperitoneal injection twice a week for 2 consecutive weeks, and the experiment ended 4 days after the last administration. Tumor volume and body weight of mice were measured and recorded twice a week. At the end of the experiment, mice were euthanized and tumor inhibition TGI was calculated. TGI (%) = [1 - (Ti - T0)/(Vi - V0)] × 100%. (Ti: the mean tumor volume of the treatment group on day i of administration, T0: the mean tumor volume of the treatment group on day 0 of administration; Vi: the mean tumor volume of the solvent control group on day i of administration, V0: the mean tumor volume of the solvent control group on day 0 of administration).

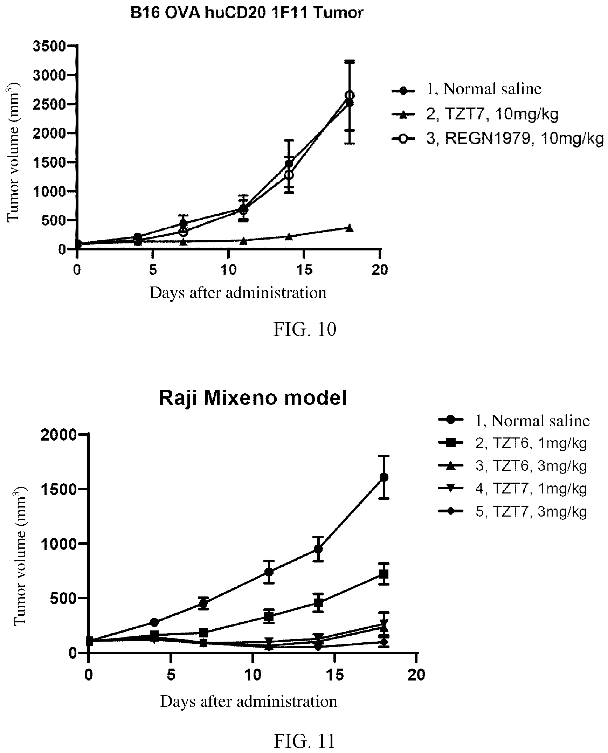

As shown in FIG. 10, on day 18 after the administration, the mean tumor volume of the normal saline control group was 2517 mm³, the mean tumor volume of the REGN1979 (Regeneron Pharmaceuticals) group was 2646 mm³, and the tumor inhibition rate was -5.3% as compared to the normal saline control group, indicating that there is no tumor inhibitory effect. The mean tumor volume of the TZT7 group was 371 mm³, and the tumor inhibition rate was 88.3% as compared to the normal saline control group, indicating that the tumor volume increase was significantly inhibited. The results showed that TZT7 could significantly inhibit the growth of a xenograft tumor B16 OVA huCD20 1F11 of hCD3e humanized mouse at a dose level of 10 mg/kg.

### Example 14. Inhibitory Effect of TZT6 and TZT7 on Human Lymphoma Raji Mixeno Model

### 1. Objective

To evaluate the anti-tumor effect of TZT6 and TZT7 of the present invention in a human lymphoma Raji Mixeno model.

### 2. Procedures

Female NDG mice (purchased from Biocytogen Jiangsu Co., Ltd.) at 6-8 weeks of old were inoculated subcutaneously on the right dorsal side with a premixed suspension (0.2 mL/mouse) of 2.5 × 10⁶ Raji cells and 5 × 10⁶ PBMC (purchased from Allcells) to establish a subcutaneous xenograft tumor model. When the mean tumor volume was about 108 mm³, appropriate animals were selected and randomly divided into 5 groups of 6 animals each based on tumor volume, which were
a solvent control group:
   - G1 normal saline group; and
treatment groups:
   - G2 TZT6 (1 mg/kg) group;
   - G3 TZT6 (3 mg/kg) group;
   - G4 TZT7 (1 mg/kg) group; and
   - G5 TZT7 (3 mg/kg) group;

All groups were subjected to intraperitoneal injection in a single dose, and the experiment ended 18 days after the last administration. Tumor volume and body weight of mice were measured and recorded twice a week. At the end of the experiment, mice were euthanized and tumor inhibition TGI was calculated. TGI (%) = [1 - (Ti - T0)/(Vi - V0)] × 100%. (Ti: the mean tumor volume of the treatment group on day i of administration, T0: the mean tumor volume of the treatment group on day 0 of administration; Vi: the mean tumor volume of the solvent control group on day i of administration, V0: the mean tumor volume of the solvent control group on day 0 of administration).

As shown in FIG. 11, on day 18 after the start of the administration, the mean tumor volume of the normal saline control group was 1610 mm³, the mean tumor volume of TZT6 was 723 mm³ at a dose level of 1 mg/kg and 233 mm³ at a dose level of 3 mg/kg, and the tumor inhibition rates were 58.7% and 91.5%, respectively, as compared to the normal saline control group, indicating that the tumor volume increase was significantly inhibited. The mean tumor volume of TZT7 was 264 mm³ at a dose level of 1 mg/kg and 99 mm³ at a dose level of 3 mg/kg, and the tumor inhibition rates were 89.6% and 100.6%, respectively, indicating that the tumor volume increase was significantly inhibited. The results showed that TZT6 and TZT7 could significantly inhibit the growth of a subcutaneous xenograft tumor of human lymphoma Raji cells at dose levels of 1 mg/kg and 3 mg/kg.

## Claims

1. A bispecific antibody specifically binding to CD20 and CD3, comprising:
(1) an antigen-binding fragment having a CD20 binding domain, comprising a first heavy chain variable region (VH1) and a first light chain variable region (VL1); and
(2) an antigen-binding fragment having a CD3 binding domain, comprising a second heavy chain variable region (VH2) and a second light chain variable region (VL2).

2. The bispecific antibody according to claim 1, comprising the following polypeptide chains:
(1) polypeptide chains of formula (I) and formula (II) having a CD20 binding domain:
VH1-CH1-Fc1 formula (I),
and
VL1-CL formula (II);
and
(2) polypeptide chains of formula (III) and formula (II-2) having CD20 and CD3 binding domains:
VH1-CH1-L-VL2-L-VH2-L-Fc2 formula (III),
and
VL1-CL formula (II-2);
or
a polypeptide chain of formula (IV) having a CD3 binding domain:
VL2-L-VH2-L-Fc2 formula (IV);
wherein VH represents a heavy chain variable region; VL represents a light chain variable region; Fc comprises CH2 and CH3; CH1, CH2, and CH3 represent domains 1, 2, and 3 of the heavy chain constant region, respectively; VL represents a light chain variable region; CL represents a light chain constant region; L represents a linker peptide, the linker peptides being identical or different; optionally, a hinge region is present between CH1 and Fc1;
formulas (I), (II), (II-2), (III), and (IV) are each formed in a sequential connection from N-terminus to C-terminus;
an antigen-binding site formed by VH1 and VL1 binds to CD20, and an antigen-binding site formed by VL2 and VH2 binds to CD3.

3. The bispecific antibody according to claim 1 or 2, wherein the linker peptide comprises an amino acid sequence (GGGGS)n, wherein n is each independently selected from 1, 2, 3, 4, 5, and 6.

4. The bispecific antibody according to any one of claims 1-3, wherein:
VH1 comprises HCDR1, HCDR2, and HCDR3 having amino acid sequences set forth in SEQ ID NO: 1, SEQ ID NO: 2, and SEQ ID NO: 3, respectively, and
VL1 comprises LCDR1, LCDR2, and LCDR3 having amino acid sequences set forth in SEQ ID NO: 4, SEQ ID NO: 5, and SEQ ID NO: 6, respectively.

5. The bispecific antibody according to claim 4, wherein:
VH1 comprises an amino acid sequence set forth in SEQ ID NO: 7, or an amino acid sequence having at least 90%, 91%, 92%, 93%, 94%, 95%, 96%, 97%, 98%, or 99% identity to the amino acid sequence set forth in SEQ ID NO: 7; and
VL1 comprises an amino acid sequence set forth in SEQ ID NO: 8; or comprises an amino acid sequence having at least 90%, 91%, 92%, 93%, 94%, 95%, 96%, 97%, 98%, or 99% identity to the amino acid sequence set forth in SEQ ID NO: 8.

6. The bispecific antibody according to any one of claims 1-3, wherein:
VH2 comprises HCDR1, HCDR2, and HCDR3 having amino acid sequences set forth in SEQ ID NO: 9, SEQ ID NO: 10, and SEQ ID NO: 11, respectively, and
VL2 comprises LCDR1, LCDR2, and LCDR3 having amino acid sequences set forth in SEQ ID NO: 12, SEQ ID NO: 13, and SEQ ID NO: 14, respectively.

7. The bispecific antibody according to claim 6, wherein:
VH2 comprises an amino acid sequence set forth in SEQ ID NO: 15, or an amino acid sequence having at least 90%, 91%, 92%, 93%, 94%, 95%, 96%, 97%, 98%, or 99% identity to the amino acid sequence set forth in SEQ ID NO: 15; and
VL2 comprises an amino acid sequence set forth in SEQ ID NO: 16, or an amino acid sequence having at least 90%, 91%, 92%, 93%, 94%, 95%, 96%, 97%, 98%, or 99% identity to the amino acid sequence set forth in SEQ ID NO: 16.

8. The bispecific antibody according to any one of claims 1-3, wherein:
the polypeptide chain of formula (I) comprises an amino acid sequence set forth in SEQ ID NO: 17 or 23, or an amino acid sequence having at least 90%, 91%, 92%, 93%, 94%, 95%, 96%, 97%, 98%, or 99% identity to the amino acid sequence set forth in SEQ ID NO: 17 or 23; and
the polypeptide chains of formula (II) and formula (II-2) comprise an amino acid sequence set forth in SEQ ID NO: 18, or an amino acid sequence having at least 90%, 91%, 92%, 93%, 94%, 95%, 96%, 97%, 98%, or 99% identity to the amino acid sequence set forth in SEQ ID NO: 18.

9. The bispecific antibody according to any one of claims 1-3, wherein the polypeptide chain of formula (III) comprises an amino acid sequence set forth in SEQ ID NO: 19, or an amino acid sequence having at least 90%, 91%, 92%, 93%, 94%, 95%, 96%, 97%, 98%, or 99% identity to the amino acid sequence set forth in SEQ ID NO: 19.

10. The bispecific antibody according to any one of claims 1-3, wherein the polypeptide chain of formula (IV) comprises an amino acid sequence set forth in SEQ ID NO: 20, or an amino acid sequence having at least 90%, 91%, 92%, 93%, 94%, 95%, 96%, 97%, 98%, or 99% identity to the amino acid sequence set forth in SEQ ID NO: 20.

11. The bispecific antibody according to any one of claims 1-3, comprising the polypeptide chains of formula (I) and formula (II), and the polypeptide chains of formula (III) and formula (II-2), wherein the polypeptide chain of formula (I) comprises an amino acid sequence set forth in SEQ ID NO: 23, the polypeptide chains of formula (II) and formula (II-2) comprise amino acid sequences set forth in SEQ ID NO: 18, and the polypeptide chain of formula (III) comprises an amino acid sequence set forth in SEQ ID NO: 19.

12. The bispecific antibody according to any one of claims 1-3, comprising the polypeptide chains of formula (I) and formula (II), and the polypeptide chain of formula (IV), wherein the polypeptide chain of formula (I) comprises an amino acid sequence set forth in SEQ ID NO: 17, the polypeptide chain of formula (II) comprises an amino acid sequence set forth in SEQ ID NO: 18, and the polypeptide chain of formula (IV) comprises an amino acid sequence set forth in SEQ ID NO: 20.

13. The bispecific antibody according to any one of claims 1-12, wherein formula (I) and formula (II) are connected via a disulfide bond, and formula (III) and formula (II-2) are connected via a disulfide bond; and formula (I) and formula (III) or formula (IV) are connected via a disulfide bond and a Knob into Hole structure of the CH3 domain;
preferably, Fc1 in formula (I) is knob-Fc, and Fc2 in formula (III) or formula (IV) is hole-Fc; or Fc1 in formula (I) is hole-Fc, and Fc2 in formula (III) or formula (IV) is knob-Fc.

14. The bispecific antibody according to any one of claims 1-13, wherein CH1-Fc1 in formula (I) and Fc2 in formula (III) or formula (IV) are in the form of IgG, e.g., IgG1, IgG2, IgG3, or IgG4, and/or CL in formula (II) and formula (II-2) is derived from a λ or κ chain.

15. An isolated nucleic acid, encoding any one or more of the polypeptide chains in the bispecific antibody according to any one of claims 1-14.

16. An expression vector, comprising the nucleic acid according to claim 15, wherein preferably, the expression vector is a eukaryotic expression vector.

17. A host cell, comprising the nucleic acid according to claim 15 or the expression vector according to claim 16, wherein preferably, the host cell is a eukaryotic cell, and more preferably, the host cell is a mammalian cell.

18. A method for preparing the bispecific antibody according to any one of claims 1-14, comprising culturing the host cell according to claim 16 under conditions suitable for the expression of the nucleic acid according to claim 15, and isolating the bispecific antibody from the host cell.

19. A pharmaceutical composition, comprising the bispecific antibody according to any one of claims 1-14, the nucleic acid according to claim 15, the expression vector according to claim 16, and/or the host cell according to claim 17, and a pharmaceutically acceptable carrier or excipient.

20. Use of the bispecific antibody according to any one of claims 1-14, the nucleic acid according to claim 15, the expression vector according to claim 16, the host cell according to claim 17, and/or the pharmaceutical composition according to claim 19 in the preparation of a medicament for preventing or treating cancer, wherein preferably, the cancer is selected from acute B-lymphocytic leukemia, diffuse large B-cell lymphoma, chronic lymphocytic leukemia, follicular lymphoma, non-Hodgkin's lymphoma, chronic myelocytic leukemia, and Burkitt's lymphoma.

21. A method for preventing or treating cancer in a subject, comprising administering to a subject in need thereof the bispecific antibody according to any one of claims 1-14, the nucleic acid according to claim 15, the expression vector according to claim 16, the host cell according to claim 17, and/or the pharmaceutical composition according to claim 19.

22. A method for detecting the presence of CD3 and/or CD20 in a sample using the bispecific antibody according to any one of claims 1-14.
